# EUROPEAN PATENT APPLICATION

(11) **EP 4 822 012 A1**
(43) Date of publication of application: **14.10.2026**
(21) Application number: 25170000.1
(22) Date of filing: 11.04.2025
(51) Int. Cl.: C12N 1/02, C12N 1/06, C12N 1/12, C12N 1/14, C12R 1/645, C12R 1/89

(54) **METHODS FOR PRODUCING AN EDIBLE PRODUCT FROM FUNGAL MYCELIUM FERMENTATION LIQUID AND PRODUCTS THEREOF**

(71) Applicant: Koralo GmbH, 85598 Baldham (DE)
(72) Inventor: ALBANESE, Guido, 85598 Baldham (DE); BADRINATH, Harshitha, 85598 Baldham (DE)
(74) Representative: Patentanwälte Dr. Keller, Schwertfeger Partnerschaft mbB

(57) **Abstract**

The present invention relates to methods for producing an edible product by cultivating edible fungal mycelium in a liquid nutrient medium under submerged fermentation conditions, separating the fermentation liquid from the mycelium biomass, and further processing this liquid into an edible product. Furthermore, the invention relates to an edible product based on fermentation liquid produced by cultivating edible fungal mycelium, preferably together with a microalgae or macroalgae strain. The products that are obtainable by the methods of the invention can be used as a drug or food supplement, e.g. for improving a subject's health, particularly for the prevention or treatment of conditions associated with impaired immune response, epithelial integrity, or for irritated mucosa tissue.

## Description

### Field of the Invention

The present invention relates to methods of producing an edible product from fungal mycelium fermentation liquid. The edible product of the invention can be used as an animal feed, feed supplement, human food supplement and/or drug with health-enhancing properties. The invention further relates to fermentation liquid that is obtainable by cultivating edible fungal mycelium, preferably in combination with microalgae or macroalgae, and further processing of the fermentation liquid into edible products. The edible products have the potency to enhance immune response, improve epithelial integrity and/or promote wound healing.

### State of the Art

A larger and more prosperous world population demands more animal protein from land-based animal husbandry and aquaculture. Traditional animal protein sources for feed, such as wild-caught fish, are declining and thus becoming more expensive. This forces a switch to a more plant-based diet for animal feed. Plant-based nutrition and feed cause digestive problems lowering feed efficiency and disrupt the gut epithelium of omnivores/carnivores, making them more vulnerable to opportunistic infections. Additionally, with larger population densities weakened or sick individuals will lead to overall higher mortality rates. That said, ensuring their recovery from dietary, biological or physical stress is key for animal rearing. As in particular mucosal tissues such as in intestines is affected, their protection and recovery are of critical importance.

Traditionally, solid feeds have been fermented to improve the digestibility of the resulting animal feed with the help of bacteria, especially lactic acid bacteria, as described in US2021/0400996 A1. Bacteria convert the solid materials and remain in the feed.

Similarly, feed additives are produced by fermentation, such as microalgae as an additive for aquafeed or yeasts to promote gut health. In these cases, the microalgae, bacteria or yeasts are grown in a submerged fermentation and then physically separated from the fermentation liquor.

Other fermentation-based feed additives relate to single substances such as amino acids or vitamins. To this end, fungi or bacteria are genetically modified to produce these single substances, grown in a fermentation liquor, and the released single substances are isolated from the fermentation liquor.

The situation is similar in humans: health is one of the most important factors for consumers when choosing food. Numerous products are supplemented with probiotic bacteria, especially lactic acid bacteria, which are expected to have positive effects on the microbiome and gut health due to their well-described probiotic effects. Natural, food-grade immune-boosting ingredients are currently limited to available vitamins and minerals, which are ingested either through their natural food sources or through specific dietary supplements. In humans, too, new ingredients could improve the function of food as medicine for the prevention of diseases and reduce or replace the need for pharmacological treatments. A key area is epithelial health, especially of the gut mucosa (Turner et al., 2009). The gut mucosa acts on one side as a barrier to food-borne diseases or toxic substances to pass into the body and has on the other side to ensure efficient uptake of nutrients into the body. The endothelial system benefits from a good uptake of nutrients and it can positively influence the therapeutic progress of diseases such as the prevention of high blood pressure or cardiovascular issues (Singh, V. et al. 2017).

Various products and methods based on fermented algae or fungi are known as food supplement or replacement. For example, EP4088585 A1 and WO2022238466 A1 describe a non-animal fish and seafood replacement product for human or animal consumption, comprising a composition of a fibrous mycelium mass of at least one edible fungal strain and edible microalgae or macroalgae or parts thereof. The edible microalgae or macroalgae and the fibrous mycelium mass were incubated together in a growth medium for the edible fungal strain and the solid mass further processed.

US2019254328 A1 describes an algae-based food that contains an algae-based cocoa butter, an algae-based powder or flour that contains protein and fiber, and optionally at least one flavoring agent, a sweetener or a fiber powder.

US2020060309 A1 describes a food with a fibrous structure that comprising at least one plant component of aquatic origin selected from microalgae or microalgae extracts, cyanobacteria or cyanobacteria extracts and marine fungi or marine fungi extracts of the genera *Schizochytrium* and *Auriantochytrium,* or a mixture of at least two of these components of aquatic origin and at least one other plant component.

Furthermore, a cocultivation of the algea *Chlorella vulgaris* (*C. vulgaris*) and three different fungi *Pleurotus geesteranus (P. geesteranus), Ganoderma lucidum (G. lucidum),* and *Pleurotus ostreatus (P. ostreatus)* has been described to purify biogas slurry and biogas simultaneously (Cao, W. et al., 2017).

All the known methods or products are based on the fermented mass of algae and fungi.

### Description of the Invention

It is the object of the present invention to provide alternative edible products and methods for producing a healthy, edible product by cultivation and fermentation of edible fungal mycelium.

This object is solved by a method for producing fermentation liquid that is further processed into an edible product.

The present invention is based on the surprising finding that fermentation liquid that is obtainable by cultivating edible fungal mycelium in a nutrient medium under submerged fermentation conditions, preferably together with at least one microalgae or macroalgae can be further processed to an edible food product or drug with specific health-promoting properties. In more particular it was found that fermentation liquid obtained from non-genetically modified mycelium-forming fungi alone and/or when fermented together with microalgae and/or macroalgae contributes to positive metabolic properties.

In a first aspect, the present invention relates to a method for producing an edible product comprising the following steps:
a) cultivating edible fungal mycelium in a liquid nutrient medium under submerged fermentation conditions in a bioreactor to generate a fermentation liquid,
b) separating the fermentation liquid from the mycelium biomass after completion of fermentation by filtration or centrifugation,
c) isolating and further processing of the fermentation liquid or components thereof into the edible product.

The term "edible product" as used in the context of the present invention designates animal feed or human food products, feed or food supplements or drugs that meet the standards for human and animal consumption. The edible product is based on the fermentation liquid or components thereof isolated by the methods described herein.

The selected "edible fungal mycelium" or "edible microalgae or macroalgae" are non-toxic for human or animal consumption, edible quantities assumed.

Cultivating edible fungal mycelium in a liquid nutrient medium under submerged fermentation conditions in a bioreactor is a controlled process that is used to efficiently grow fungal biomass for subsequently isolating the desired fermentation liquid. In a first step, an edible fungal species is selected. Ideally, the strain should be high-yielding, fast-growing and nutritionally rich. The nutrient medium must support rapid mycelial growth and can contain a carbon source (e.g., glucose, sucrose, starch, molasses), nitrogen source (e.g., ammonium sulfate, yeast extract, peptone), minerals (e.g., magnesium, potassium, phosphorus), vitamins and trace elements (for optimal metabolism). Usually, the pH is adjusted to be around 4 to 8 for fungal growth. A small volume of fungal inoculum (e.g. from a pre-culture) is introduced into the bioreactor. Any suitable bioreactor can be used such as stirred-tank reactor, air-lift reactor or bubble column reactor so that the fungus grows in the liquid medium, forming mycelial filaments rather than fruiting bodies. Growth can be controlled by modifying or controlling the aeration and oxygen supply, agitation, temperature, pH. If necessary, antifoaming agents can be added if foam production during fermentation is excessive. Examples of such antifoaming agents include, but are not limited to silicone-based antifoams, oil-based antifoams, polyoxyethylene/polypropylene-based antifoams, alcohol-based antifoams, or natural antifoams such as lecithin or saponins. Typically, after 15 to 250 hours, the biomass can be harvested, and filtered or centrifuged to separate the fungal mycelium from the desired fermentation liquid. The biomass is not further required and can be disposed of.

The choice of separation method depends on the viscosity, particle size, and density of the biomass. Preferred separation methods include, but are not limited to filtration, centrifugation, sedimentation, membrane separation or decantation. In some embodiments, vacuum filtration is used, i.e. a vacuum pump to pull liquid through a filter membrane or cloth, leaving mycelial biomass behind. Alternatively, pressure filtration or rotary drum filters can be utilized. For finer mycelial suspensions, batch centrifugation or continuous centrifugation is preferred.

Removal of residual biomass and impurities can be carried out by filtration or centrifugation. It can also be necessary to adapt the pH to optimize the stability of the product. Activated carbon or enzymatic treatment can be applied to remove pigments, if desired. The liquid can be concentrated by evaporation, ultrafiltration or reverse osmosis. In some cases, the edible product (e.g. food product or drug) can be pasteurized or sterilized for further use. In some embodiments, the edible product can be freeze-dried. Depending on the product, flavor enhancers, stabilizers, or natural preservatives can be added to the final product. In some embodiments, the product can be converted into powder form by applying spray-drying, freeze-drying or drum-drying methods. For end use, the product can be provided as a liquid drink, as a squeezable paste or as a powder for food supplement.

The edible product has positive metabolic properties including, but not limited to antitumor, antiviral, antibacterial, antidiabetic or antihypercholesterolic properties. As demonstrated herein, one property of the edible product obtained from fermentation liquid is that it has an antioxidant effect. A second property is that it enhances the immune response and a third one that it strengthens the epithelium integrity. In addition, the fermentation liquid can positively contribute to epithelial health by regeneration of mucosa tissue after an insult, in particular mucosa intestinal tissue. The effect of the edible product according to the present invention can be boosted by further additives such as minerals, vitamins or probiotic microorganisms. If used in therapeutic applications, the edible product can be a medicament.

In more preferred embodiments, the methods according to the present invention further comprise cultivating the edible fungal mycelium together with at least one microalgae or macroalgae strain, and separating the mycelium biomass and algae from the fermentation liquid. It was surprisingly found that co-cultivation of the fungal mycelium together with microalgae or macroalgae enhances the nutritional profile and bioactivity of the resulting edible product.

In preferred embodiments, co-cultivating the edible fungal mycelium with at least one microalgae or macroalgae strain in a liquid nutrient medium is carried out under submerged fermentation conditions, preferably in a bioreactor. Separating the fermentation liquid from the mycelium and algal biomass after completion of fermentation is preferably carried out by filtration or centrifugation. As described above for the other embodiments, the fermentation liquid can then be isolated and further processed into the edible product. Further processing of the fermentation liquid that is obtained by co-cultivation of mycelia with micro or macro algae to the edible product comprises any one of precipitation, evaporation, distillation, filtration, centrifugation, adsorption, reverse osmosis, extraction, solid-phase microextraction, chromatography, freeze-drying, electrolysis, dialysis, or combinations thereof, allowing for various concentrations and formulations of the active components present in the fermentation liquid.

In some embodiments, the fermentation liquid can be added directly to the food, feed, or food supplement or feed supplement. If necessary, the fermentation liquid can be concentrated. In some embodiments, the isolated and purified fermentation liquid can be added to a food or feed product in concentrations that boost the general health conditions (prophylactic) or treat negative health conditions (therapeutic) in a subject. Alternatively, the fermentation liquid can be further processed into a drug that can be administered as a therapeutic agent to a patient suffering from a disease to be treated.

A "subject" as used herein relates to an animal or human, fed or treated with an edible product according to the present invention. In some embodiments, the edible product is a drug or medicament that is suitable in the prevention or treatment of a medical condition or disease that is associated with impaired immune response or impaired epithelial integrity. In addition, the drug or medicament produced from a fermentation liquid according to the present invention can promote the recovery of epithelial tissue, in particular mucosal tissue. An increased epithelial permeability is associated with Inflammatory bowel disease (IBS) and other inflammatory conditions. The epithelial system (lining of the gut mucosa) is closely linked to the endothelial system (lining of the blood vessels) in the intestine controlling nutrition exchange, fluid balance, and immune responses. Damage or dysfunctions such as inflammations in one layer can significantly impact the other. Therefore, a damage or inflammation of the gut epithelium can negatively affect endothelial health leading to cardiovascular conditions such as diabetes, heart failure, heart attacks or strokes and mental conditions such as anxiety or depression. To prevent the above medical conditions the edible product prophylactically maintains epithelium integrity and boost immune responses in both epithelial and endothelial systems. In case of damage of the epithelial tissue the edible product therapeutically boosts immune response and speeds up recovery of integrity of the epithelium.

Diseases or conditions that are associated with an impaired immune response include, but are not limited to immunodeficiency disorders, autoimmune diseases, chronic inflammatory diseases or infectious diseases.

Diseases or conditions that are associated with an impaired epithelial integrity include, but are not limited to inflammatory bowel disease (IBD), celiac disease, asthma and chronic obstructive pulmonary disease (COPD), respiratory infections, atopic dermatitis, cystic fibrosis, psoriasis, graft-versus-host disease, necrotizing enterocolitis (NEC), sepsis or cancer.

Diseases or conditions that are associated with mucosa tissue damage include, but are not limited to respiratory tract mucosal damage such as asthma, allergic rhinitis, COPD, or gastrointestinal tract mucosal damage such as IBD, celiac disease, IBS or NEC, or oral and nasopharyngeal mucosa damage.

Stimulating immune response will indirectly improve the outcome of other diseases such as bacterial and viral infections as well as tumors.

In animal feed in particular the edible product stimulates gut homeostasis leading to healthier animals with higher feed conversion and growth rates. Protein to fat ratio is improving as is mortality rates.

Any suitable fast-growing fungal strain can be used for the production of the edible product as long as it produces sufficient metabolites released into the fermentation liquid. In preferred embodiments, the edible fungal mycelium is derived from the phylum Basidiomycota and/or Ascomycota.

In some preferred embodiments, the edible fungal mycelium is derived from at least one fungal strain selected from the group consisting of *Pleurotus ostreatus, Pleurotus sapidus, Pleurotus pulmonarius, Ganoderma lucidum, Hericium erinaceus, Inonotus obliquus, Trametes versicolor, Grifola fondosa, Lentinula edodes, Aspergillus oryzae, Rhizopus oryzae, Fusarium graminareum, Cordyceps militaris, Cordyceps sinensis, Tuber melanosporum, Tuber magnatum, Neurospora intermedia, Neurospora sitophila,* and *Xylaria hypoxion.* Each of these strains contributes unique metabolites to the fermentation liquid.

In embodiments in which macro or micro algae are co-cultivated with fungal mycelium, at least one microalgae or macroalgae strain is preferably selected from a wide range of species, including, but not limited to *Chlorella vulgaris, Chlamydomonas reinhardtii, Haematococus pluvialis, Dunaliella salina, Scenedesmus dimorphus, Scenedesmus acuminatus, Tetraselmis suecica, Tetraselmis striatis, Arthrospira platensis, Synechococcus sp., Isochrysidus galbana, Nitzschia dissipata, Phaedactylum tricornutum, Skeletonema sp., Pseudonana sp., Odontella aurita, Schizochytrium sp., Aurantiochytrium sp., Monodus subterranea, Nannochloropsis gaditana, Nannochloropsis oceanica, Nannochloropsis oculata, Porphyridium cruentum, Pyropia yezoensis, Pyropia haitianensis, Palmaria palmata, Palmaria mollis, Chondrus crispus, Kapaphycus alvarezii, Eucheuma cottonii, Ulva lactuca, Sacharina japonica, Sacharina latissima,* and *Laminaria digitata.* The co-cultivation of the fungal mycelium with microalgae or macroalgea provides additional nutrients and bioactive compounds to the fermentation process.

In some aspects of the invention, the methods for producing the edible product may further comprise the steps of
- harvesting and subjecting the algal biomass to a cell disruption treatment after cultivation, wherein the disruption treatment comprises any one of physical, chemical, or enzymatic treatment,
- transferring the disrupted algal biomass to a second bioreactor containing edible fungal mycelium, and
- cultivating the edible fungal mycelium with the disrupted algal biomass in a nutrient medium under submerged fermentation conditions before separating the fermentation liquid from the mycelium biomass.

This treatment allows enhanced extraction of beneficial compounds from the algae and the formation of different metabolic compounds from edible fungi mycelium.

The nutrient medium used in the methods of the invention preferably comprises a carbon source, a nitrogen source, phosphates, sulphates, and/or minerals, providing the necessary nutrients for optimal growth and metabolite production. The fermentation process may be performed as a batch, fed-batch or continuous process, allowing for flexibility in production scale and efficiency. The product preferably further comprises one or more additives selected from the group consisting of minerals, vitamins, and/or probiotic microorganisms to enhance its nutritional and functional properties. The edible product can be then used in a variety of ways as animal feed, feed supplement, human food supplement and/or drug.

In one aspect, the product obtained by the methods of the invention can be used as a drug or feed/food supplement with the property to enhance positive metabolic effects. These can result in an enhanced immune response, improved epithelial integrity and/or promoted wound healing.

There are three different basic variants of the methods according to the invention. In a first variant, the feed or food supplement is obtainable by fermentation of the edible mycelium on its own. This means the mycelium from edible fungi is grown either in a batch, a fed batch or a continuous fermentation process inside a bioreactor. The mycelium is supplemented with suitable micro- and macro-nutrients and maintained at ideal conditions like temperature, pH, and oxygen availability for a sufficient period of time for its optimal growth. The mycelium is then harvested from the bioreactor and separated from the supernatant or residual fermentation liquid by using techniques like filtration or centrifugation. The residual fermentation liquid is rich in metabolites or bio active compounds produced by the mycelium which benefit other animals or humans. Therefore, the residual fermentation liquid, after the separation of the mycelium, is further cleaned for any residual mycelium or organic matter and either used as such or subjected to additional processes like freeze-drying, spray-drying, electrolysis, dialysis or any combination of the aforementioned methods to be ready for use as animal feed, or feed or food supplement. If required, the fermentation liquid can be enriched with additional nutrient-rich components.

In a second variant, the mycelium from edible fungi and microalgae or macroalgae are synergistically grown in the same bioreactor. The mycelium and the co-cultivated microalgae or macroalgae mutually benefit from each other by providing favorable growth conditions to their co-living organism with their symbiotic association. For example, microalgae or macroalgae initially break down complex nutrients in the media which can be metabolized by the mycelium. In addition, O₂ availability to the mycelium is increased, while the mycelium provides CO₂ to enhance the growth of microalgae or macroalgae. Furthermore, both types of organisms are supplemented with any additional micro and macro nutrients, and grow under ideal conditions, which leads to optimal yield. Preferably, co-fermentation takes place inside a bioreactor in a batch, fed batch or continuous process, with minor customization of the reactor such as incorporating LED light for the microalgae or macroalgae. After fermentation, the mycelial biomass along with the flocculated microalgae or macroalgae on the surface of the mycelia are harvested as described herein and the residual fermentation liquid is used as such or subjected to further process like freeze-drying or spray-drying or electrolysis or dialysis or any combination of the mentioned methods to be used for the animal feed as such or feed or food supplement, optionally in combination with additional nutritionally rich components.

In a third variant, any edible microalgae or macroalgae or a combination of multiple edible microalgae or macroalgae is subjected to physical or chemical or enzymatical or any combination of the aforementioned processes to disrupt the cell structures prior to transferring them into the main bioreactor containing the edible fungal mycelium. The disrupted microalgae or macroalgae boost the mycelium fermentation process by providing prime nutrients like carbon, nitrogen sources and additional vitamins for enhanced growth of the mycelium. Furthermore, it enhances the omega 3 fatty acids in the final fermentation media which otherwise would be partly absorbed by the biomass or left in the fermentation residue. The disrupted microalgae or macroalgae must be free of microbial contamination before adding it into the main reactor, which is ensured, for instance, by heat treatment as known to the skilled person. In addition to the disrupted microalgae or macroalgae cells, other missing micro- and macro-nutrient requirements are supplemented to the main bioreactor in which the fermentation takes place. The fermentation is carried out in a batch, fed batch or continuous process under ideal conditions with controlled temperature, pH and oxygen availability for a sufficient period of time. After fermentation, the final harvest is performed as described herein, and residual fermentation liquid is used as such or is subjected to further processes like freeze-drying, spray-drying, or electrolysis or dialysis or any combination of the aforementioned methods to be used as animal fee, feed or food supplement, optionally in combination with additional nutritionally rich components.

In a fourth variant, the residual fermentation media is subjected to second fermentation with a known concentration of aerobic probiotic bacteria in a stir tank bioreactor or inside a disinfected large container. The probiotic bacteria such as but not limited to *Lactobacillus acidophilus, Bifidobacterium lactis,* or *Streptococcus thermophilus* or a combination thereof can utilize or metabolize the residual or unutilized sugars or carbon sources, nitrogen sources and other trace minerals left in the residual fermentation media obtained from first, second and third embodiments. To enhance the bioactivity of the added probiotics, the process is optimized by adjusting pH, temperature and aeration. The resulting outcome of the process results in liquid containing the metabolites or bioactive compounds produced by the mycelium and probiotic bacteria which has the potential for antioxidant property, immunity enhancement, epithelial integrity, wound healing properties and probiotic properties in humans and fish or any livestock. Therefore, the secondary fermentation liquid is used as such without any further processing. In some embodiments, the liquid is either centrifuged to limit the concentration of the probiotic bacteria or filtered with filters to separate the probiotic bacteria. Preferred filters have a pore size of 22 microns. As an alternative or in addition to the aforementioned, the liquid can be subjected to further processes like freeze-drying, spray-drying, electrolysis or dialysis, or any combination of the before mentioned methods to produce the animal feed or feed supplement, or food supplement. In some embodiments, the feed or food supplement can be combined with additional nutritionally rich feed components.

In some embodiments, the microalgae or macroalgae are first cultivated or grown separately in a bioreactor under the most ideal conditions possible in order to achieve optimum growth, which is then aseptically transferred to the main reactor where co-fermentation takes place.

In all embodiments, the residual liquid obtained as a reaction product from the fermentation is suitable for animal feed or human food, animal feed or human food supplement, or drug, which can accelerate the immune response, the integrity of the epithelium and wound healing due to the bioactive compounds contained therein. As shown here, the residual fluid from fermentation has the properties of improving immune response, epithelial integrity and faster wound healing under both prophylactic and therapeutic conditions.

Macroalgae as described in this invention can be all non-toxin producing species, preferably those used for food consumption. If macroalgae are selected from the division Rhodophyta, the classes of Bangiophyceae and of Florideophyceae are preferred. Within the class of Bangiophyceae, the order Bangiales and within that order the family Bangiaceae and within that family the genus *Pyropia* are preferred and most preferred are the species *Yezoensis* and *Haitianensis.*

If macroalgae are selected from the class of Florideophyceae then within that class the order Palmariales and Gigartinales are preferred. If the order Palmariales is selected, the family Palmariaceae and within that family the genus *Palmaria* are preferred and most preferred the species *Palmata* and *Mollis.* If the order Gigartinales is selected, the family Gigartinaceae and Solieriaceae are preferred. Within the family Gigartinaceae the genus *Chondrus* are preferred and most preferred the species *Crispus.* Within the family Solieriaceae the genus *Kapaphycus* and *Eucheuma* are preferred. If the genus *Kapaphycus* is selected, *Alvarezii* is the most preferred species. If the genus *Eucheuma* is selected, *Cottonii* is the most preferred species.

If macroalgae are selected from the division Chlorophyta, the class of Ulvophyceae and within that class the order Ulvales and within that order the family Ulvaceae and within that family the genus *Ulva* are preferred and most preferred are the species *Lactuca.*

If macroalgae are selected from the division Ochrophyta, the class of Phaeophyceae and within the class the order Laminariales and within that order the family Laminariaceae and within that family the genus *Sacharina* and *Laminaria* are preferred. If the genus *Sacharina* is selected, the most preferred are the species J*aponica* and *Latissima.* If the genus *Laminaria* is selected, the most preferred are the species *Digitata.*

As microalgae all non-toxin producing species are suitable and in general microalgae consumed as food or used for food ingredients are preferred.

If the microalgae are selected from the division Chlorophyta the class of Trebouxiophyceae, Chlorodentrophyceae and Chlorophyceae are preferred. If the class of Trebouxiophyceae is selected, the order Chlorellales is preferred. If the order Chlorellales is selected, the family Chlorellaceae is preferred.

If the family Chlorellaceae is selected, the genus *Chlorella* is preferred and most preferred is the species *Vulgaris.*

If the class of Chlorophyceae is selected, the order Chlamydomonadales and Sphaeropleales are preferred. If the order Chlamydomonadales is selected, the families of Haematococaceae, Dunaliellaceae and Chlamydomonadaceae are preferred. If the family Chlamydomonadaceae is selected, the genus *Chlamydomonas* is preferred and most preferred is the species *Reinhardtii.* If the family of Haematococaceae is selected, the genus *Haematococus* is preferred and most preferred is the species *Pluvialis.* If the family of Dunaliellaceae is selected, the genus *Dunaliella* is preferred and most preferred is the species *Salina.*

If the order Sphaeropleales is selected, the family of Scenedesmaceae is preferred. If the family Scenedesmaceae is selected, the genus *Scenedesmus* is preferred and most preferred are the species *Dimorphus* and *Acuminatus.*

If the class of Chlorodentrophyceae is selected, the order Chlorodendrales is preferred. If the order Chlorodendrales is selected, the family Chlorodendraceae is preferred. If the family Chlorodendraceae is selected, the genus *Tetraselmis* is preferred and most preferred are the species *Suecica* and *Striatis.*

If the microalgae are selected from the division cyanobacteria, the class of Cyanophyceae is preferred. Within the class of Cyanophyceae, the order Oscillatoriales and Synechococcales are preferred. If the order Oscillatoriales is selected, the family Microcoleaceae is preferred. If the family Microcoleaceae is selected, the genus *Arthrospira* is preferred and most preferred is the species *Platensis.* If the order Synechococcales is selected, the family Synechococcaceae is preferred. If the family Synechococcaceae is selected, the genus *Synechococcus* is preferred.

If the microalgae are selected from the division Haptophyta, the class of Prymnesiophyceae and Pavlovophyceaea are preferred. If the class of Prymnesiophyceae is selected, the order Isochrysidales is preferred. If the order Isochrysidales is selected, the family Isochrysidaceae is preferred. If the family Isochrysidaceae is selected, the genus *Isochrysidus* is preferred and most preferred is the species *Galbana.* If the class of Pavlovophyceaea are selected, the order Pavlovales is preferred. If the order Pavlovales is selected, the family Pavlovaceae is preferred.

If the microalgae are selected from the division Ochrophyta the class of Bacillariophyceae, Coscinodiscophyceae, Labyrinthulomycetes, Xantophyceae and Eustigmatophyceae are preferred. If the class of Bacillariophyceae is selected, the order Bacillariales is preferred. If the order Bacillariales is selected, the family Bacillariaceae and Phaeodactylaceae are preferred. If the family Bacillariaceae is selected, the genus *Nitzschia* is preferred and most preferred is the species *Dissipata.* If the family Phaeodactylaceae is selected, the genus *Phaedactylum* is preferred and most preferred is the species *Tricomutum.*

If the class of Coscinodiscophyceae is selected, the order Thalassiosirales and Biddulphiineae are preferred. If the order Thalassiosirales is selected, the family Skeletonemataceae and Thalassiosiaceae are preferred. If the family Skeletonemataceae is selected, the genus *Skeletonema* is preferred. If the family Thalassiosiaceae is selected, the genus *Pseudonana* is preferred.

If the order Biddulphiineae is selected, the family Eupodiscaceae is preferred. If the family Eupodiscaceae is selected, the genus *Odontella* is preferred and most preferred is the species *Aurita.*

If the class of Labyrinthulomycetes is selected, the order Labyrinthulales is preferred. If the order Labyrinthulales is selected, the family Thraustochytriaceae is preferred. If the family Thraustochytriaceae is selected, the genus *Schizochytrium* and *Aurantiochytrium* is preferred.

If the class of Xantophyceae is selected, the order Mischocodcales is preferred. If the order Mischocodcales is selected, the family Pleurochloridaceae is preferred. If the family Pleurochloridaceae is selected, the genus *Monodus* is preferred and most preferred is the species *Subterranea.*

If the class of Eustigmatophyceae is selected, the order Eustigmatales is preferred. If the order Eustigmatales is selected, the family Monodopsidaceae is preferred. If the family Monodopsidaceae is selected, the genus *Nannochloropsis* is preferred and most preferred are the species *Gaditana, Oceanica* and *Oculata.*

If the microalgae are selected from the division Rhodophyta, the class of Porphyridiophyceae is preferred. If the class of Porphyridiophyceae is selected, the order Porphyridiales is preferred. If the order Porphyridiales is selected,the family Porphyridiaceae is preferred. If the family Porphyridiaceae is selected, the genus *Porphyridium* is preferred and most preferred is the species *Cruentum*.

The mycelium used in the present invention originates from at least one fungal strain, bacterial strain or a combination thereof. The mycelium is derived from heterotrophic organisms requiring organic material and oxygen for growth. The mycelium is the vegetative part of a fungus or fungus like bacterial colony, consisting of a mass of branching cells. As such it is an extension of the hypha of fungi that emanates from a fungal spore. The fungus is for example a filamentous fungus.

The fungus is for example selected from the phylum Basidiomycota, Ascomycota, Glomeromycota, Mucoromycota, Zoopagomycota or a combination thereof.

Further, the fungus is for example selected from the division agaricomycotina, ustilagomycotina, pezizomycotina, saccharomycotina, taphrinomycetes, diversisporalis, archaeosporales, paraglomerales, endogonales, mucorales, mortieralles, entomophthoromycotina, asellariales, kickxellales, dimargaritales, harpellales, zoopagomycotina, or a combination thereof.

Moreover, the fungus is for example selected from the class tremellomycetes, dacrymycetes, agaricomycetes, exobasisiomycetes,ustilaginomycetes, malasseziomycetes, moniliellomycetes, arthoniomycetes, coniocybomycetes, dothideomycetes, eurotiomyctes, geoglossomycetes, laboulbeniomycetes, lecanoromycetes, leotiomycetes, lichinomycetes, orbiliomycetes, pezizomycetes, sordariomycetes, xylonomycetes, or a combination thereof.

Furthermore, the fungus is for example selected from the order filobasidiales, agaricales, amylocorticiales, atheliales, boletales, jaapiales, lepidostromatales, geastrales, gomphales, hysterangiales, phallales, auriculariales, cantherellales, corticiales, gleophylalles, hymenochaetales, polyporales, russulales, sebacinales, stereopsidales, thelephorales, trechisporales, ceraceosorales,doassansiales, entyomatales, exobasidiales, georgefischeriales, microstromatales, tilletiales, urocystales, ustilaginales, malassezioales, moniliellales, saccharomycetales, coronophorales, glomeralles, Hypocreales, melanosporales, microascales, boliniales, calosphaeriales, chaetospheriales, coniochaetales, diasporthales, magnaporthales, ophiostomatales, sordariales, xylariales, koralionastetales, lulworthiales, meliolales, phylachoralles, trichosphariales, eurotiales, chaetothyriales, pyrenulales,verrucariales, onygenales, mortierellales, mucorales, endogonales, or combinations thereof.

Alternatively, the fungus is selected from the family *Filobasidium, Dacromycetaceae, Agaricaceae, Amanitaceae, Bolbitiaceae, Broomeiceae, Chromocyphellaceae, Clavariaceae, Cortinariaceae, Cyphellaceae, Enolomataceae, Fistulinaceae, Himigasteraceae, Hydnangiaceae, Hygrophoraceae, Inocybaceae, Limnoperdacea, Lyophyllaceae, Marasmiaceae, Mycenacea, Niaceae, Pellorinaceae, Physalacriaceae, Pleurotacea, Pluteaceae, Porotheleaceae, Psathyrellaceae, Pterulacea, Schizophyllaceae, Stephanosporaceae, Strophariaceae, Tricholomataceae, Typhulaceae, Boletaceae, Boletinellaceae, Coniophoraceae, Diplocystaceae, Gasterellaceae, Gastrosporiaceae, Gomphidiaceae, Gyroporaceae, Hygrophoropsidaceae, Paxillaceae, Protogastraceae, Rhizopogonaceae, Sclerodermataceae, Serpulaceae, Suillaceae, Tapinellaceae, Hymenochaetaceae, Repetobasidiaceae, Schizoporaceae, Cystostereaceae, Fomitopsidaceae, Fragiporiaceae, Ganodermataceae, Gelatoporaceae, Meripilaceae, Merulaciaea, Phenerochaetaceae, Polyporaceae, Sparassidaceae, Steccherinaceae, Xenasmataceae, Albatrellaceae, Amylostereaceae, Auriscalpaceae, Bondarzewiaceae, Echinodontiaceae, Hericiaceae, Hybogasteraceae, Lachnocladiaceae, Peniphoraceae, Russulaceae, Gloeocyctidiellacceae, Stereaceae, Ustilaginomycetes, Saccharomycetaceae, Saccharomycodaceae, Saccharomycopsidaceae, Chaetomiaceae, Lasiosphaeriaceae, Sordariaceae,* or combinations thereof.

Further, the fungus is selected from the genus *Neurospora, Aspergillus, Trichoderma, Pleurotus, Ganoderma, Inonotus, Cordyceps, Ustilago, Rhizopus, Tuber, Fusarium, Pennicillium, Xylaria, Trametes, Lentinula, Hericium, Grifola* or a combination thereof.

Furthermore, the fungus is selected from the group consisting of *Pleurotus ostreatus, Pleurotus sapidus, Pleurotus pulmonarius, Ganoderma lucidum, Hericium erinaceus, Inonotus obliquus, Trametes versicolor, Grifola fondosa, Lentinula edodes, Aspergillus oryzae, Rhizopus oryzae, Fusarium graminareum, Cordyceps militaris, Cordyceps sinensis, Tuber melanosporum, Tuber magnatum, Neurospora intermedia, Neurospora sitophila,* and *Xylaria hypoxion* or combinations thereof.

Edible fungi are preferably fungal strains selected from the division Basidiomycota or Ascomycota.

If the division of Basidiomycota is selected, the preferred subdivision is Agaromycotina. If the subdivision Agaromycotina is selected, the preferred class is Agaricomycetes.

If the class Agaricomycetes is selected, the order Agaricales, Auriculariales, Boletales, Cantharellales, Polyporales and Russulales are preferred.

If the order Boletales is selected, the families *Boletaceae* and *Sclerodermataceae* are preferred.

If the order Cantharellales is selected, the families *Cantharellaceae* and *Hydnaceae* are preferred.

If the order Agaricales is selected, the families *Agaricaceae, Fistulinaceae, Lyophyllaceae, Marasmiaceae, Omphalotaceae, Physalacriaceae, Pleurotaceae, Schizophyllaceae, Strophariaceae,* and *Tricholomataceae* are preferred.

If the order Polyporales is selected, the families *Ganodermataceae, Meripilaceae, Polyporaceae,* and *Sparassidaceae* are preferred.

If the order Russulales is selected, the families *Bondarzewiaceae* and *Hericiaceae* are preferred.

If the order Auriculariales is selected, the family *Auriculariaceae* is preferred.

If the family *Pleurotaceae* is selected, the genus *Pleurotus* is preferred and most preferred is the species *Pulmonarius.*

If the division Ascomycota is selected, the subdivision Pezizomycotina is preferred. If the subdivision Pezizomycotina is selected, the classes Pezizomycetes and Sordariomycetes are preferred.

If the class Pezizomycetes is selected, the order Pezizales is preferred. If the order Pezizales is selected, the families *Morchellaceae* and *Tuberaceae* are preferred.

If the class Sordariomycetes is selected, the order Hypocreales and Sordariales are preferred.

If the order Hypocreales is selected, the families *Cordycipitaceae* and *Nectriaceae* are preferred. If the order Sordariales is selected, the family *Sordariaceae* is preferred.

In preferred embodiments, the edible product obtainable by the methods of the invention is a drug because of its therapeutic potential. In one aspect of the present invention, an edible product obtainable by the methods described herein is provided for use in health enhancement comprising the prevention or treatment of a disease or condition that is associated with impaired immune response, epithelial integrity or regeneration of mucosa tissue, specifying the health-promoting applications.

In a preferred embodiment, the edible product is part of a pharmaceutical composition. The edible product is obtainable by any of the methods described therein. In one aspect, the edible product comprises the fermentation liquid. In other aspects, the edible product can also be components or ingredients that have been isolated from the fermentation liquid obtained by the methods of the invention. In some embodiments, the components that form the edible product are chemicals, intermediates or proteins isolated from the fermentation liquid. If specific components are used these will be produced from the edible product by precipitation, evaporation, distillation, filtration, centrifugation, adsorption, reverse osmosis, extraction, solid-phase microextraction, chromatography, freeze-drying, spray-drying, drum-drying, electrolysis, dialysis, or combinations thereof, allowing for various concentrations and formulations of the active components present in the edible product.

Useful components include, but are not limited to beta-glucans (immunomodulatory), cordycepin (anti-inflammatory, antiviral), ergothioneine (antioxidant), or polysaccharide-protein complexes (immune support). Polysaccharides may need to be enzymatically broken down or ultrasonicated for better dispersion. To obtain a stable aqueous solution or suspension, the isolated compound can be dissolved in sterile saline or water (e.g. 0.9% NaCl) or dispersed with surfactants or stabilizers (e.g., polysorbate 80, glycerol). Parameters such as pH (ideally 6-7.4 for use as an aersol), osmolarity (match body fluids ~300 mOsm/kg), sterility or microbial load (critical for inhalables) and particle size (< 5 microns if suspended) must be checked.

The pharmaceutical composition may be formulated with any known pharmaceutically acceptable carrier or diluent, as well as any other known adjuvants and excipients, in accordance with conventional techniques. The pharmaceutically acceptable carriers, diluents, adjuvants and excipients should be suitable for the chosen modulator of the present invention and the chosen mode of administration. A pharmaceutical composition of the present invention may also include diluents, fillers, salts, buffers, detergents (e.g., a nonionic detergent), stabilizers (e.g., sugars or protein-free amino acids), preservatives, tissue fixatives, solubilizers, and/or other materials suitable for inclusion in a pharmaceutical composition.

The pharmaceutical compositions comprising the edible product or components thereof of the present invention can be formulated by methods known to those skilled in the art. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of the fermentation liquid is adjusted.

The edible product is preferably administered to a subject either by consumption, oral administration or through nasal administration, preferably as an aerosol.

To obtain an aerosol, preferably a nebulizer is used that converts the liquid edible product (provided as a medication or solution) into a fine mist (aerosol) that can be inhaled by the individual into the lungs. This is especially useful for respiratory treatments. Ideal for respiratory deposition are 1 to 5 microns.

### Description of the Figures

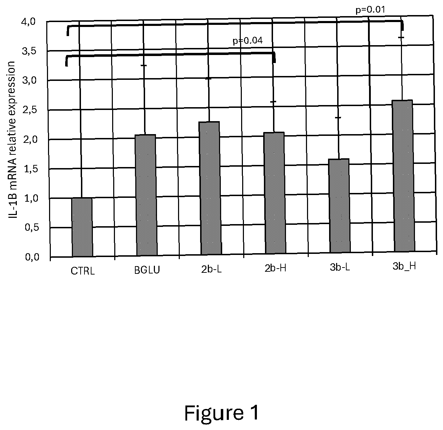

Figure 1. Relative expression of the immune-related molecular markers, Interleukin 1Beta (IL 1B) in intestinal explants after co incubated with test ingredients 2b, 3b, at low (-L Low) and high (-H High) concentration. CTRL indicates unexposed explant, and BGLU indicates commercial β Glucan. Bars indicate mean ± SE, and horizontal lines indicate significant differences (ANOVA, Tukey-HSD post-hoc, p<0.05) (Ingredient details are presented in Table 1).

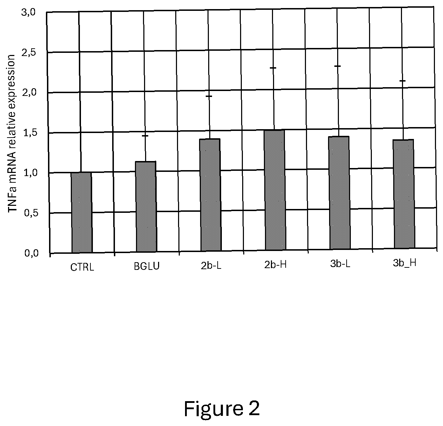

Figure 2. Relative expression of the immune-related molecular markers, Tumor necrosis factor alpha (TNF-alpha) in intestinal explants after co incubated with test ingredients 2b, 3b, at low (-Low) and high (-High) concentration. CTRL indicates unexposed explant, and BGLU indicates commercial β Glucan. Bars indicate mean ± SE, and horizontal lines indicate significant differences (ANOVA, Tukey-HSD post-hoc, p<0.05) (Ingredient details are presented in Table 1).

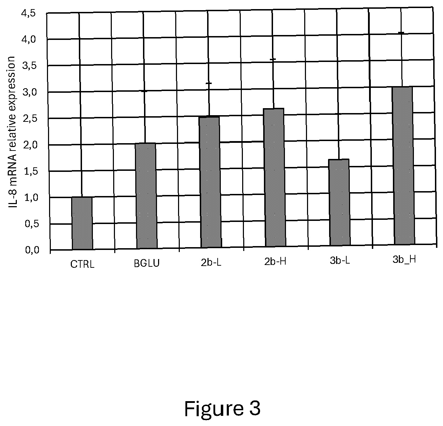

Figure 3. Relative expression of the immune-related molecular markers, Interleukin 8 (IL-8), in intestinal explants after co incubated with test ingredients 2b, 3b, at low (-L Low) and high (-H High) concentration. CTRL indicates unexposed explant, and BGLU indicates commercial β Glucan. Bars indicate mean ± SE, and horizontal lines indicate significant differences (ANOVA, Tukey-HSD post-hoc, p<0.05) (Ingredient details are presented in Table 1).

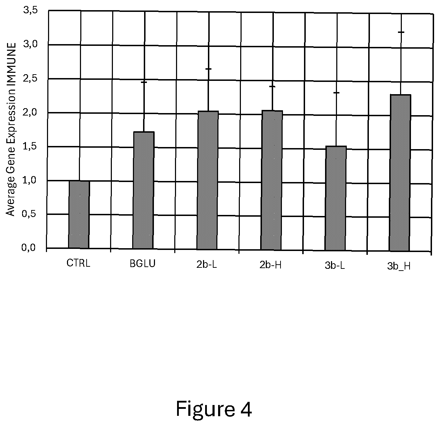

Figure 4. Average relative expression of the immune-related molecular markers, in intestinal explants after co-incubated with test ingredients 2 and 3b, at low (- L Low) and high (-H High) concentration. CTRL indicates unexposed explant and BGLU indicates commercial β-Glucan. Bars indicate mean ± SE and there were no significant differences (ANOVA, p<0.05) (Ingredient details are presented in Table 1).

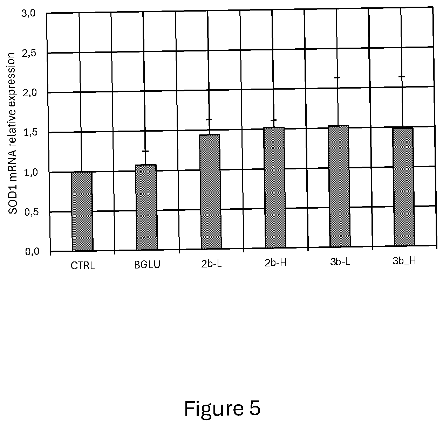

Figure 5. Relative expression of the antioxidant response-related molecular markers, Superoxide dismutase 1 (SOD1) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

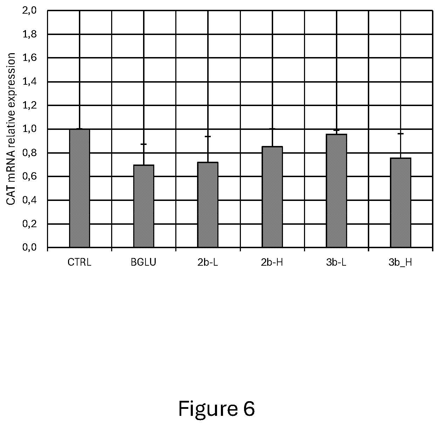

Figure 6. Relative expression of the antioxidant response-related molecular markers, Catalase (CAT) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

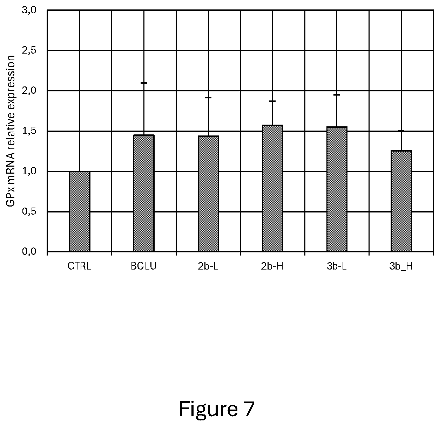

Figure 7. Relative expression of the antioxidant response-related molecular markers, Glutathione peroxidase (GPx), in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

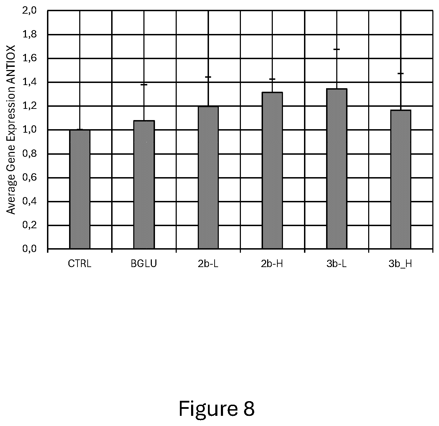

Figure 8. Average relative expression of the antioxidant response-related molecular markers, in intestinal explants after co-incubated with test ingredients 2b, 3b, at low (-L Low) and high (-H High) concentration. CTRL indicates unexposed explant and BGLU indicates commercial β-Glucan. Bars indicate mean ± SE and there were no significant differences (ANOVA, p>0.05) (Ingredient details are presented in Table 1).

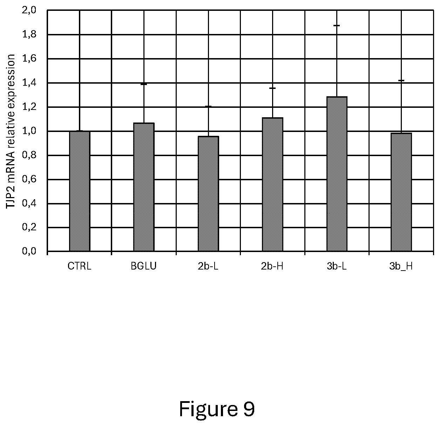

Figure 9. Relative expression of the epithelium integrity response-related molecular markers, Tight junction protein 2 (TJP2) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

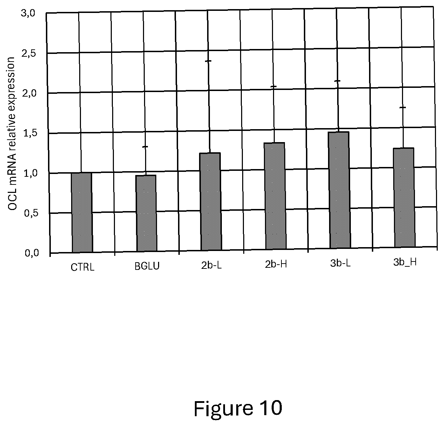

Figure 10. Relative expression of the epithelium integrity response-related molecular markers, Occludin (OCL) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

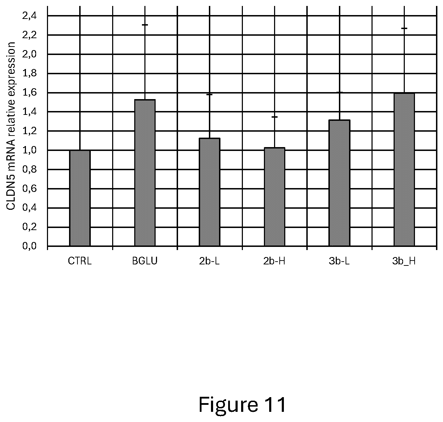

Figure 11. Relative expression of the epithelium integrity response-related molecular markers, Claudin 5 (CLDN5) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

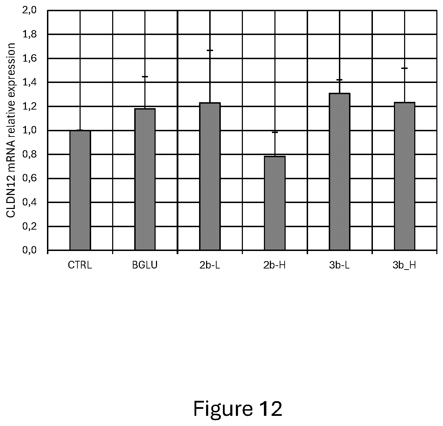

Figure 12. Relative expression of the epithelium integrity response-related molecular markers, Claudin 12 (CLDN12) in intestinal explants after co-incubated with test ingredients 2b, 3b, at low (-L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

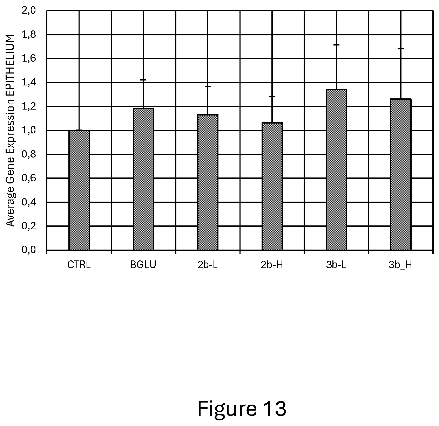

Figure 13. Average relative expression of the epithelium integrity response-related molecular markers, in intestinal explants after co-incubated with test ingredients 2b, 3b, at low (- L Low) and high (-H High) concentration. CTRL indicates unexposed explant, and BGLU indicates commercial β-Glucan. Bars indicate mean ± SE, and there were no significant differences (ANOVA, p>0.05) (Ingredient details are presented in Table 1).

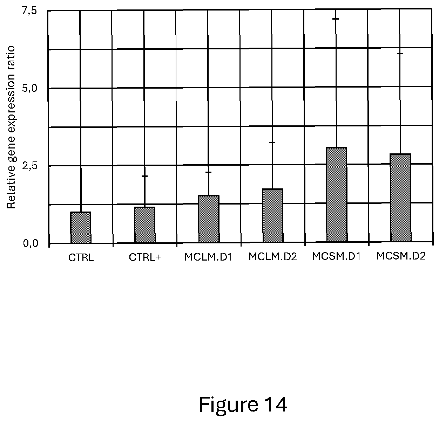

Figure 14. Relative expression of the cyclooxygenase 2 (cox-2), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

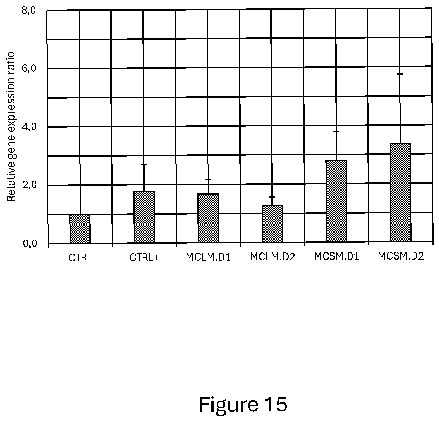

Figure 15. Relative expression of the Interleukin 8 (IL-8), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

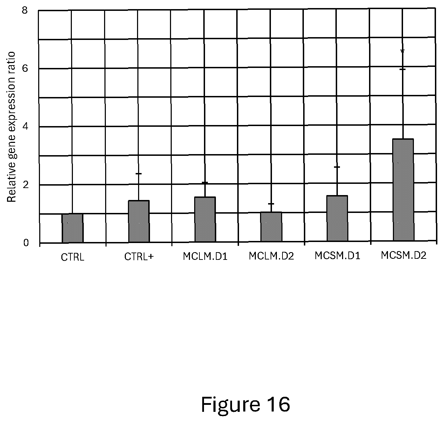

Figure 16. Relative expression of the Interleukin 1 Beta (IL-1B), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV, and asterisk indicate significant differences from CTRL (Dunnet test, p<0.05)

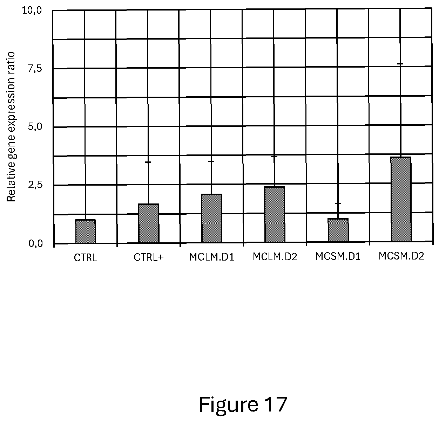

Figure 17. Relative expression of the tumor necrosis factor alpha (TNF-alpha), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

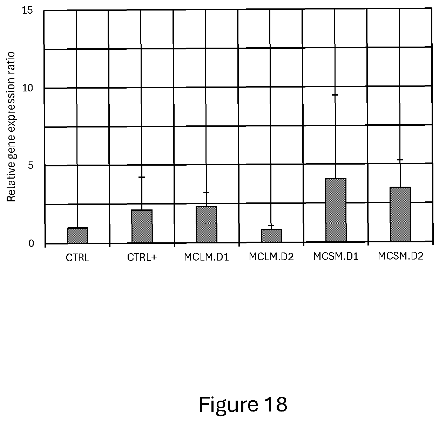

Figure 18. Relative expression of the immunoglobulin M (IgM), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

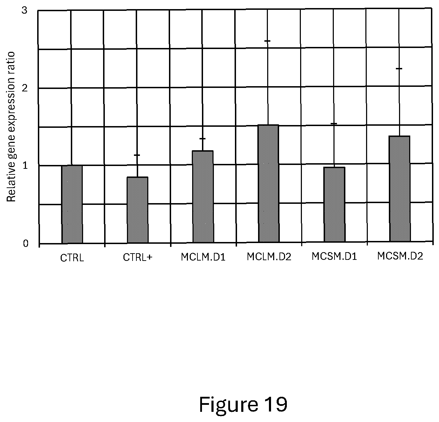

Figure 19. Relative expression of claudin12 (cldn12), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

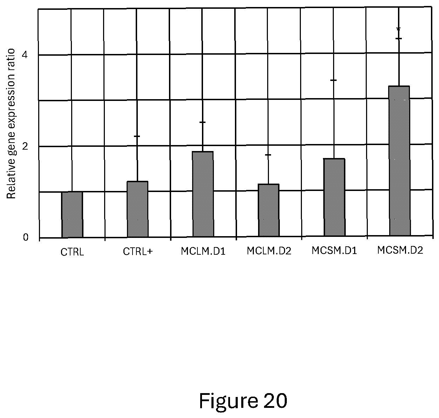

Figure 20. Relative expression of claudin5 (cldn5), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV, and asterisk indicate significant differences from CTRL (Dunnet test, p<0.05).

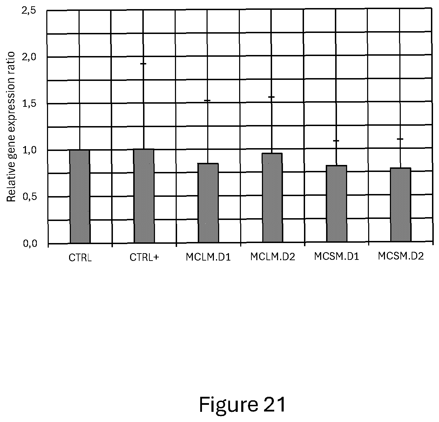

Figure 21. Relative expression of occludin (ocl), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

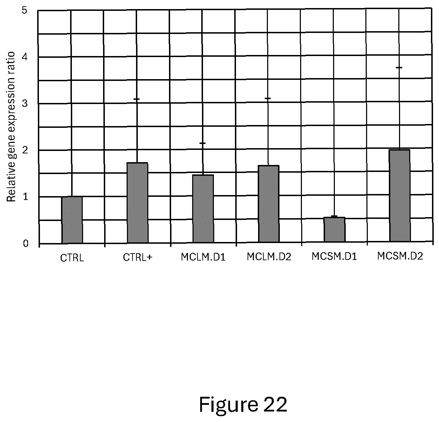

Figure 22. Relative expression of intestinal mucin (imuc), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

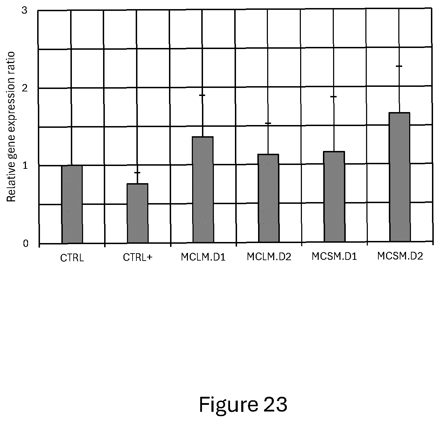

Figure 23. Relative expression of tight junction protein (tjp), in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

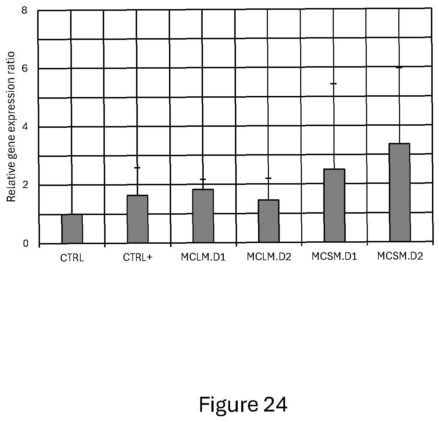

Figure 24. Mean relative expression of all immune related genes, in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

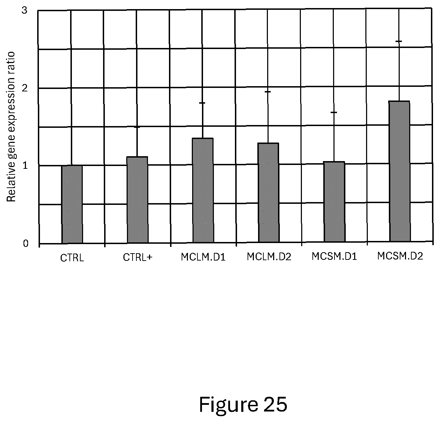

Figure 25. Mean relative expression of all epithelium integrity related genes, in intestinal explants after incubation with test ingredients MCLM and MCSM at low (D1) and high (D2) concentration. CTRL indicates unexposed explant, and CTRL+ indicates commercial β-Glucan. Bars indicate mean ± SDEV.

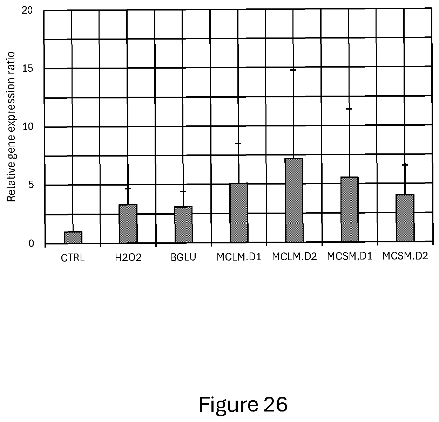

Figure 26. Relative expression of cycloxygenase2 (COX-2), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV.

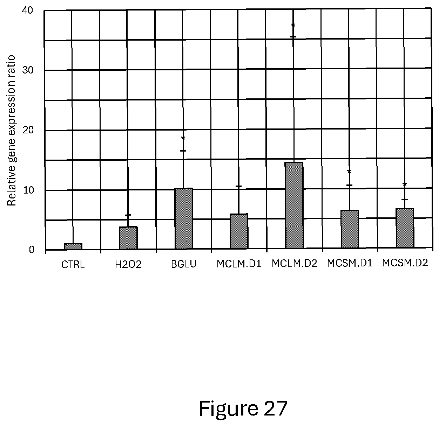

Figure 27. Relative expression of interleukin 1 beta (IL-1B), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV, and asterisks indicate significant differences with CTRL (Dunnet's test, p<0.05).

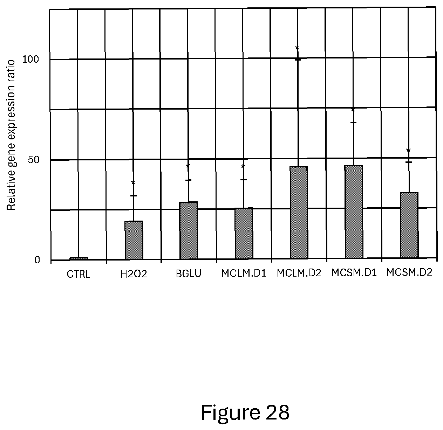

Figure 28. Relative expression of interleukin 8 (IL-8), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV, and asterisks indicate significant differences with CTRL (Dunnet's test, p<0.05).

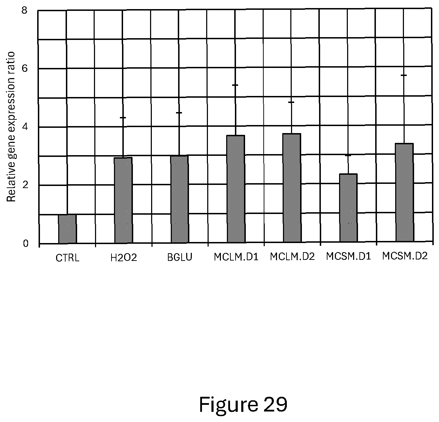

Figure 29. Relative expression of tumor necrosis factor alpha (TNF-alpha), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV.

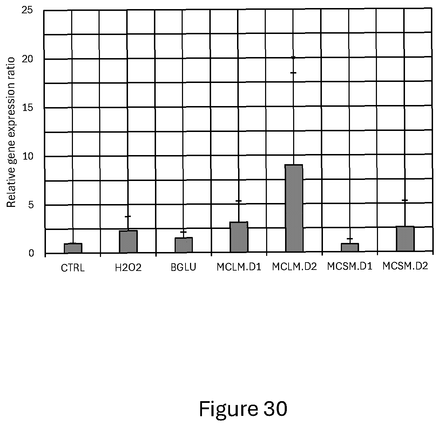

Figure 30. Relative expression of immunoglobulin M (IgM), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV, and asterisks indicate significant differences with CTRL (Dunnet's test, p<0.05).

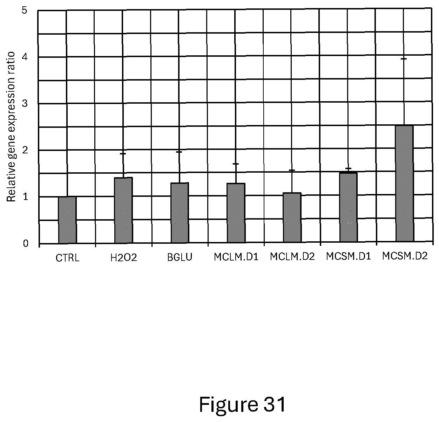

Figure 31. Relative expression of claudin 12 (CLDN12), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV.

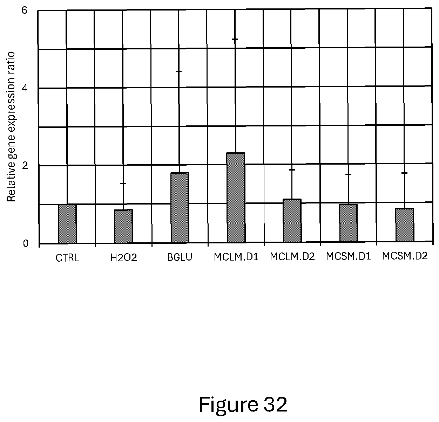

Figure 32. Relative expression of claudin 5 (CLDN5), in intestinal explants throughout an insult with an irritant (H2O2) and incubation with test ingredients MCLM and MCSM at low (D1) and high (D2) concentration. CTRL indicates unexposed explant, H2O2 indicates tissue with insult and BGLU indicates incubation with β-Glucan. Bars indicate mean ± SDEV.

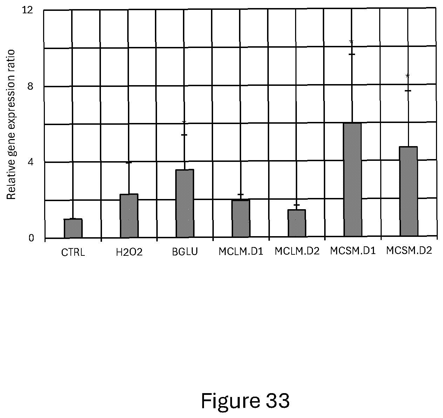

Figure 33. Relative expression of occludin (OCL), in intestinal explants throughout an insult with an irritant (H2O2) and incubation with test ingredients MCLM and MCL at low (D1) and high (D2) concentration. CTRL indicates unexposed explant, H2O2 indicates tissue with insult and BGLU indicates incubation with β-Glucan. Bars indicate mean ± SDEV, and asterisks indicate significant differences with CTRL (Dunnet's test, p<0.05).

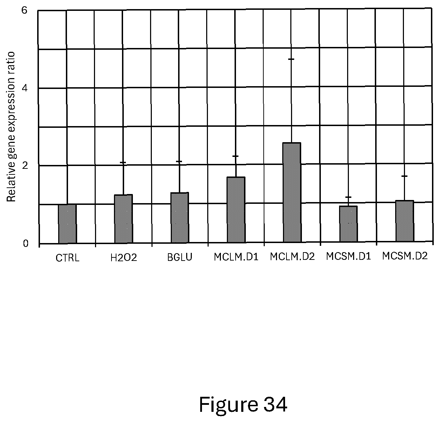

Figure 34. Relative expression of tight junction protein 2 (TJP2), in intestinal explants throughout an insult with an irritant (H2O2) and incubation with test ingredients MCLM and MCL at low (D1) and high (D2) concentration. CTRL indicates unexposed explant, H2O2 indicates tissue with insult and BGLU indicates incubation with β-Glucan. Bars indicate mean ± SDEV.

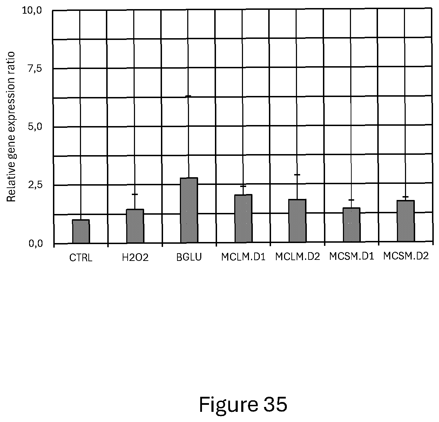

Figure 35. Relative expression of intestinal mucins (iMUC), in intestinal explants throughout an insult with an irritant (H2O2) and incubation with test ingredients MCLM and MCL at low (D1) and high (D2) concentration. CTRL indicates unexposed explant, H2O2 indicates tissue with insult and BGLU indicates incubation with β-Glucan. Bars indicate mean ± SDEV.

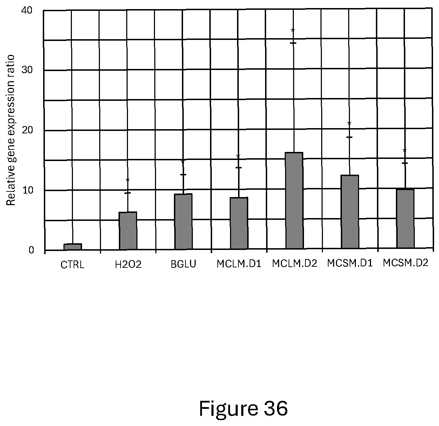

Figure 36. Mean relative expression of the immune related genes in intestinal explants throughout an insult with an irritant (H2O2) and incubation with test ingredients MCLM and MCL at low (D1) and high (D2) concentration. CTRL indicates unexposed explant, H2O2 indicates tissue with insult and BGLU indicates incubation with β-Glucan. Bars indicate mean ± SDEV, and asterisks indicate significant differences with CTRL (Dunnet's test, p<0.05).

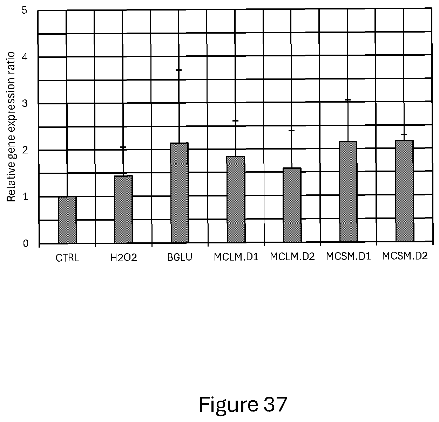

Figure 37. Mean relative expression of epithelium integrity related genes in intestinal explants throughout an insult with an irritant (H2O2) and incubation with test ingredients MCLM and MCL at low (D1) and high (D2) concentration. CTRL indicates unexposed explant, H2O2 indicates tissue with insult and BGLU indicates incubation with β-Glucan. Bars indicate mean ± SDEV.

### EXAMPLES

The present invention is further illustrated in the following examples. By no means shall the invention be limited to those specific embodiments. The invention also comprises combinations of individual features or entire embodiments described herein

### Example 1: Cultivation of Microalgae

*Aurantiochytrium acetophilum* and *Chlorella vulgaris* obtained from GreenColab, Portugal were grown on F2 nutrient media. The composition of the F2 media is: 0.003 g/L sodium phosphate (NaH₂PO₄), 0.075 g/L sodium nitrate (NaNO₃), 0.03 g/L sodium silicate (Na₂SiO₃), 1mL/L trace elements and 0.5mL/L vitamin mix. The trace elements contain 3.15 g/L FeCl₃·6H₂O, 4.36 g/L Na₂EDTA·2H₂O, 0.0098 g/L CuSO₄·5H₂O, 0.0063 g/L Na₂MoO₄·2H₂O, 0.022 g/L ZnSO₄·7H₂O, 0.01 g/L CoCl₂·6H₂O, 0.18 g/L MnCl₂·4H₂O, where each of the components is dissolved separately in distilled water and combined at the end. The vitamin mix contains 200 mg/L thiamine HCl (vit. B1), 1 mg/L biotin (vit. H), and 1 mg/L cyanocobalamin (vit. B12), where each of the components is dissolved separately in distilled water and combined later.

The cryopreserved algae vials of *Aurantichytrium acetophilum* and *Chlorella vulgaris* were taken out from liquid nitrogen and thawed. The respective cultures were inoculated into 5L IKA benchtop bioreactors equipped with an agitator for uniform mixing, along with innovative marine 2 in 1 14W LED light and incubated at 30°C and 200 rpm agitation for 3-4 days. The reactor was harvested and the respective cultures were collected in clean 5L bottles and closed with a cap. The collected culture was centrifuged at 10000 rpm for 10 minutes to separate them from the unutilized media or supernatant and stored in the fridge to be used when required.

### Example 2: Cultivation of the Mycelium

### Pre-culture

The *Pleurotus pulmonarius* mycelium vial purchased from Mycelinarium, was initially grown on potato dextrose agar for 7-8 days at 28°C in an IKA INC 125 digital incubator. As the mycelium grows fully like a lawn on the agar plate, a square piece of 1×1 cm along with agar was cut from the center of the plate under aseptic condition and transferred to a 250mL Erlenmeyer flask containing 100mL of the growth medium and a few glass beads. The growth medium contains 25 g/L glucose, 2.5 g/L peptone, 2 g/L yeast extract, 0.6 g/L KH₂PO₄, 0.4 g/L K₂HPO₄, 0.5 g/L MgSO₄, 0.5 g/L NaCl, 0.05 g/L CaCl₂, adjusted at pH 6.5 before sterilization with 1M HCl or 1M NaOH solution (Ahlborn, J et al., 2019). The flask was incubated at 28-30°C in dark under static state for 7-8 days. The mycelium again grew as a lawn on the surface of the medium. The mycelium lawn was further broken into tiny pieces with glass beads by vigorous shaking of the flask. The obtained mycelium suspension was used to inoculate a 1L Erlenmeyer flask containing the same media composition and incubated at 28-30°C on an IKA INC 125 FS digital shaker incubator at 150 rpm for 7-8 days.

### Seed Culture

A 10L stir tank IKA habitat double wall bioreactor with 6L working volume containing the same media used for the pre-cultures was further supplemented with a trace solution of 4mL/L, where the trace solution contains 80 mg/L FeCl₃·6H₂O, 90 mg/L ZnSO₄·7H₂O, 30 mg/L MnSO₄·H₂O, 5 mg/L CuSO₄·5H₂O, 400 mg/L EDTA and adjusted the pH to 6.5 with 1M HCl or 1M NaOH (Trapp, T et al., 2017). The reactor was sterilized along with the media according to the SIP programme built in with the software of the reactor. The reactor was then inoculated with 5% inoculum obtained from the pre-culture. The fermentation was carried out at 28°C at 150 rpm agitation in absence of light and with dissolved oxygen maintained up to 70-80% saturation throughout for 6-7 days.

### Main Mycelium Fermentation

The seed culture was transferred or scaled to the 1 cubic custom-made stir tank BiostatSTR bioreactor from Sartorius with 600L working volume containing the same media used for seed culture. The fermentation is carried out at 28°C at 150 rpm agitation in absence of light and with dissolved oxygen maintaining 70-80% saturation throughout for 6-7 days. Following fermentation, the reactor was harvested and the harvest was collected in clean containers. The collected harvest was filtered using a muslin cloth to separate the mycelium biomass from the supernatant or the residual fermentation liquid to obtain a non-turbid liquid free of solid matter. The residual fermentation liquid was collected separately and frozen immediately in a shock freezer from Thermo Scientific^{™} XBF40D -40°C Blast Freezers from Thermo Fisher Scientific. The separated mycelium biomass was washed in de-ionized water two to three times. The washed mycelium biomass and the collected frozen residual fermentation liquid were freeze dried in a Harvest Right Home Pro medium freeze drier from Trimleaf for further ex-vivo studies and chemical analysis.

**Table 1:**

| **Trials No** | **Fermentation of edible fungal mycelium** | **Volume of the residue fermentation liquid(L)** | **Sample code used in Ex-vivo studies** |
|---|---|---|---|
| 1 | Edible fungal mycelium | 450 | MCSM |

### Example 3: Co-fermentation of Edible Fungal Mycelium with Aurantiochytrium acetophilum

*Aurantiochytrium acetophilum* was cultured under heterotrophic conditions on F2 media in a 10L stir tank bioreactor with 6L final working volume similar to as explained in example 1. The *Aurantiochytrium acetophilum* was grown at 30°C at 200rpm agitation for 3-4 days. Post incubation, the reactor was only partially harvested under clean condition without contaminating the remaining 0.8L *Aurantiochytrium acetophilum* culture that was retained in the bioreactor.

In trial 1 the remaining culture of the *Aurantiochytrium acetophilum* was supplemented with 800 mL of sterilized media which contains 25 g/L glucose, 2.5 g/L peptone, 2 g/L yeast extract, 0.6 g/L KH₂PO₄, 0.4 g/L K₂HPO₄, 0.5 g/L MgSO₄, 0.5 g/L NaCl, 0.05 g/L CaCl₂ and 4mL/L trace solution, where the trace solution contains 80 mg/L FeCl₃·6H₂O, 90 mg/L ZnSO₄·7H₂O, 30 mg/L MnSO₄·H₂O, 5 mg/L CuSO₄ 5H2O, 400 mg/L EDTA and adjusted the pH to 6.5 with 1M HCL or 1M NaOH. To this 5% v/v of edible fungal mycelium pre-culture of example 2 was transferred under aseptic conditions. The fermentation was carried out at 28°C at 150 rpm agitation in the absence of light and with dissolved oxygen maintained up to 80-90% saturation throughout for 6-7 days.

Following fermentation, the reactor was harvested and the harvest was collected in clean containers as sample 2b.

In trial 2 300 mL of the harvested *Aurantiochytrium acetophilum* culture were added to 5.7 L of sterilized media which contains 25 g/L glucose, 2.5 g/L peptone, 2 g/L yeast extract, 0.6 g/L KH₂PO₄, 0.4 g/L K₂HPO₄, 0.5 g/L MgSO₄, 0.5 g/L NaCl, 0.05 g/L CaCl₂ and 4mL/L trace solution, where the trace solution contains 80 mg/L FeCl₃·6H₂O, 90 mg/L ZnSO₄·7H₂O, 30 mg/L MnSO₄·H₂O, 5 mg/L CuSO₄ 5H2O, 400 mg/L EDTA and adjusted the pH to 6.5 with 1M HCL or 1M NaOH. To this 5% v/v of edible fungal mycelium pre-culture of example 2 was transferred under aseptic conditions. The fermentation was carried out at 28°C at 150 rpm agitation in the absence of light and with dissolved oxygen maintained up to 80-90% saturation throughout for 6-7 days.

Following fermentation, the reactor was harvested and the harvest was collected in clean containers as sample 3b.

The collected harvests were filtered using a muslin cloth to separate the mycelium biomass from the supernatant or the residual fermentation liquid. The residual fermentation liquid was collected separately and frozen immediately in a shock freezer. The collected frozen residual fermentation liquids were freeze dried in a freeze drier for further ex-vivo studies and chemical analysis.

**Table 2:**

| **Trial s No** | **Co-fermentation with different concentration of microalgae** | **Volume of the residue fermentation liquid(L)** | **Sample code used in Ex-vivo studies** |
|---|---|---|---|
| 1 | Edible fungal mycelium + 800mL *Aurantiochytrium acetophilum* | 1.6 | 2b |
| 2 | Edible fungal mycelium + 300mL *Aurantiochytrium acetophilum* | 6 | 3b |

### Example 4: Fermentation of edible fungal mycelium with ruptured microalgae

The harvested and stored microalgae pellets of *Chlorella vulgaris* from example 1 were transferred into a 10L stir tank bioreactor individually. The microalgae was resuspended in fermentation media that contains 25 g/L glucose, 2.5 g/L peptone, 2 g/L yeast extract, 0.6 g/L KH₂ PO₄, 0.4 g/L K₂ HPO₄, 0.5 g/L MgSO₄, 0.5 g/L NaCl, 0.05 g/L CaCl₂ and 4mL/L trace solution, where the trace solution contains 80 mg/L FeCl₃·6H₂O, 90 mg/L ZnSO₄·7H₂O, 30 mg/L MnSO₄·H₂O, 5 mg/L CuSO₄·5H₂O, 400 mg/L EDTA and adjusted the pH to 6.5 with 1M HCL or 1M NaOH and autoclaved at 121°C, 15psi pressure for 15 minutes. The sterilization of *Chlorella vulgaris* was to break or rupture its cell wall. To this 5% v/v of edible fungal mycelium pre-culture of example 2 was transferred under aseptic conditions. The fermentation was carried out at 28°C at 150 rpm agitation in the absence of light and with dissolved oxygen maintained up to 80-90% saturation throughout for 6-7 days.

Following fermentation, the reactor was harvested and the harvest was collected in clean containers. The collected harvest was filtered using a muslin cloth to separate the mycelium biomass from the supernatant or the residual fermentation liquid. The residual fermentation liquid was collected separately and frozen immediately in a shock freezer. The collected frozen residual fermentation liquid was freeze dried in a freeze drier for further ex-vivo studies and chemical analysis.

**Table 3:**

| **Trial s No** | **Co-fermentation with different concentration of microalgae** | **Volume of the residue fermentation liquid(L)** | **Sample code used in Ex-vivo studies** |
|---|---|---|---|
| 1 | Edible fungal mycelium + disrupted *Chlorella vulgaris* | 7 | MCLM |

### Example 5: Fermentation of fermentation liquid with probiotic bacteria

The residual fermentation liquid produced from the first, second and third embodiment was further subjected to a second fermentation with probiotic bacteria. A ready combination of probiotic bacteria of *Lactobacillus acidophilus, Bifidobacterium animalis* subsp *lactis, Streptococcus thermophilus* (Novonesis) was suspended in 10mL of 0.9% saline to prepare an inoculum stock and inoculated to the residual fermentation liquid at 10^6 CFU/mL final concentration. The primary residual fermentation liquid was adjusted to pH 7 with 1M NaOH before inoculation with probiotic bacteria. The secondary fermentation was carried out in a clean disinfected 25L drum with 15L working volume in a heat cabinet (Memmert 75), set to a temperature to 37°C +/- 2°C for 30h. Post incubation, the fermentation product was collected and frozen for further analysis.

### Example 6 - Feeding trial of seabass

Feeding trials of seabass can be performed to demonstrate the effect of supplemental edible product on the growth performance, feed utilization and the intestinal health status in European seabass, previously subjected to an inflammatory event with dextran sodium sulphate (DSS).

The trial comprises two experimental phases:
- Phase I: chemically-induced intestinal inflammation using a diet with 1% dextran sodium sulphate (DSS) on the overall fish population for 15 days;
- Phase II: experimental feeding of the 5 experimental feeds over 60 days, to assess their effect on the growth performance and regeneration of intestinal health criteria.

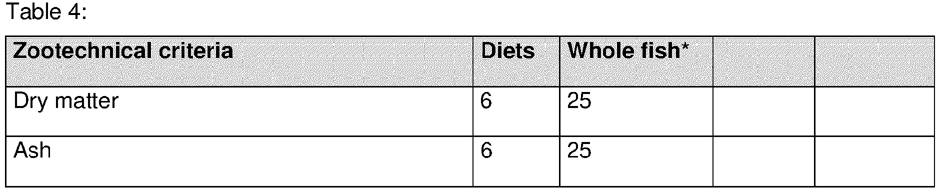

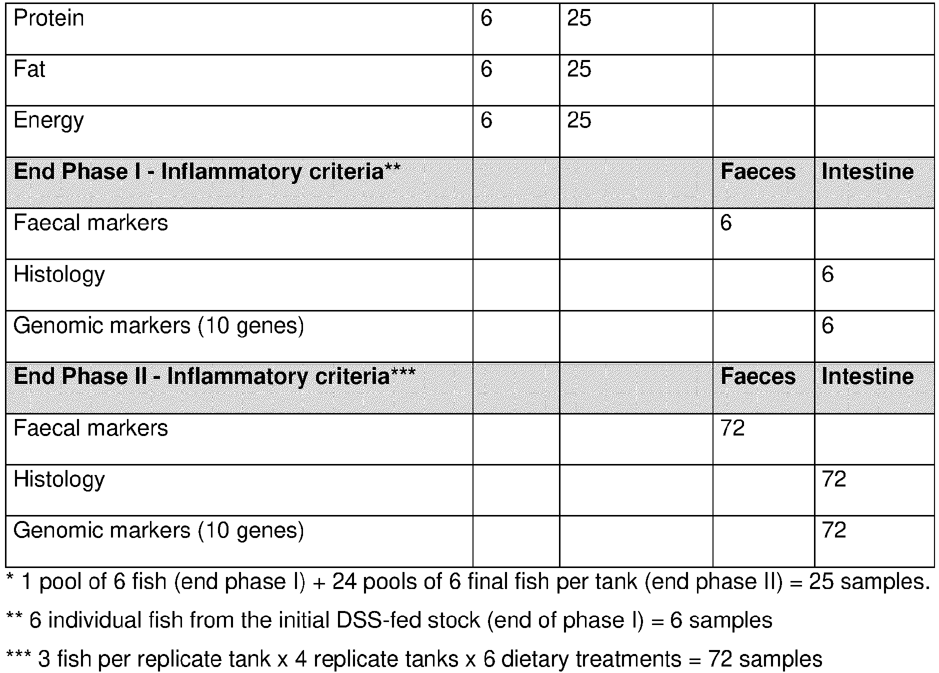

**Data generation will show:**
▪ Improved mortality
▪ Higher growth (weight gain, specific growth rate)
▪ Better feed utilization (FCR, feed intake, protein efficiency ratio)
▪ Whole-body composition: dry matter, ash, protein, fat, energy with less fat build-up
▪ Higher nutrient retention (% of intake): protein, fat, energy
▪ Improvement in faecal inflammatory markers: mucins and calprotectin
▪ Positive response in genomic markers of intestinal health (12 genes)

### Analysis of the potential of residual fermentation liquid related to the invention

### First ex-vivo study

A first ex-vivo study conducted on Seabream fish *(Sparus aurata)* with an objective to evaluate the potential of the fermentation media produced from a fermentation of fungal mycelium and micro- or macroalgae for their antioxidant property, immunity enhancement, and epithelial integrity in humans and fish or any livestock. The samples used for this experiment were obtained from example 3.

In this first study freeze dried samples of residual fermentation liquid by the fermentation process were tested. The sample 2b was obtained by co-fermentation of edible fungal mycelium and microalgae of active *Aurantiochytrium sp* in a high concentration, while sample 3b was a fermentation product of edible fungal mycelium and microalgae of active *Aurantiochytrium sp* in a high concentration.

The study uses an intestinal explant platform to assess tissue's responses to the test ingredients, to infer immune modulatory capacity, changes in antioxidant response and regulation of epithelium integrity and permeability. The evaluation of two products each in two concentrations were analyzed, and its comparison with a commercial product recognized by one of the features of interest.

**Table 5:**

| **SI No** | **Sample code** | **Sample description** |
|---|---|---|
| 1 | 2b | Freeze dried residual co-fermentation media obtained from co-fermentation of edible fungal mycelium and *Aurantiochytrium sp* (algae inoculated in high concentrations for co-fermentation) from Example 3 |
| 3 | 3b | Freeze dried residual co-fermentation media obtained from co-fermentation of edible fungal mycelium and active *Aurantiochytrium sp* (algae inoculated in low concentrations for co-fermentation) from Example 3 |

### Seabream explant trial

After the technical assessment and assurance of conformity of the test ingredients, they were placed in 2mL sterile Eppendorf tubes with culture media. Two dosages were selected following fish size to simulate a 0.15% and a 1% inclusion rate. Ingredients were prepared immediately before the start of the intestinal explant process and were always kept at the same temperature as fish were stocked, which was the incubation temperature throughout the procedure (19°C). After a preliminary assurance of good health condition, juvenile seabream *(Sparus aurata)* (mean body weight 25g) was captured with a soft net from stock facilities and anesthetized. Fish anterior intestines were used for the assay, which was performed following in-house standardized procedures. The response of fish intestines to the different ingredients and dosages was assessed after 5 hours.

### Sample handling

By the end of the trial, tissue samples were collected and placed in the RNALater (Invitrogen, USA) stabilizer solution until analysis. Samples were kept in a cold chain and stored at -80°C upon arrival at the laboratory.

### Molecular markers assessment

The tissue's total RNA was isolated using the Maxwell RSC simplyRNA Tissue Kit on a Maxwell RSC Automated extractor (Promega, USA) with slight modifications to address tissue specificities. Tissue handling was performed on ice, and RNA extraction was performed promptly to avoid degradation. RNA was quantified and purity assessed by absorbance measurement in a Nanodrop One (ThermoScientific, USA), and integrity was confirmed by electrophoresis, running samples in a MOPS 1.2% agarose gel under denaturing conditions. Complementary cDNA was synthesized by a reverse transcriptase using the RevertAid First Strand cDNA Synthesis Kit (ThermoScientific, USA) following the manufacturer's protocol. Here, 1000ng of total RNA was synthesized, and a confirmatory PCR was performed with a reference gene (EF1a) using the GoTaq Flexi DNA Polymerase (Promega, UK) with a denaturing step of 95°C for 5min, followed by 35 cycles of denaturing at 95°C for 30 sec, annealing at 60°C for 30 sec and extending at 72°C for 1min, after which a final extension took place at 72°C for 5 min.

The PCR amplicon was resolved in electrophoresis with a TAE gel 1.5%, and after running for 30 min at 110V, a photo was documented to confirm a correct amplification. The resulting cDNA was used as a DNA template for the gene expression assessment. The expression of the selected molecular markers was assessed through real-time quantitative PCR using the PowerTrack SYBR Green Master Mix for qPCR (ThermoFisher, UK) following the manufacturer's instructions in a 384-plate QuantStudio 5 device (ThermoFisher, UK). The genes Elongation factor 1a (EF1a) and the 18S ribosomal subunit (18S), were used as reference genes. Genes of interest for immune response comprised the interleukin-1beta (IL-1b), interleukin-8 (IL-8), and tumor necrosis factor-alpha (TNF-alpha). Genes of interest for antioxidant response were superoxide dismutase (SOD), glutathione peroxidase (GPx), and catalase (CAT), and the epithelial-related genes occludin (OCL), tight junction protein 2 (TJP2), claudin 12 (cldn12), and claudin 5 (cldn5). Primers for all genes were previously standardized for the tissues to assess the efficiency and correct amplification.

### Statistical analysis

Gene expression was assessed following Pfaffl's method (Pfaffl, 2001; DOI:10.1093/nar/29.9.e45). Both reference genes were used for the calculations. Data was assessed separately for each fish (biological unit) and were screened for outliers with Grubs' test. Parametric assumptions were assessed by inspection of the distribution's skewness and kurtosis (for normality), and Levene's test (for homogeneity of variance), and data were transformed with BoxCox transformation. Differences were evaluated by One-Way ANOVA followed by Tukey's post-hoc test when applicable. Multivariate analysis was performed through Discriminant Analysis to infer groups' differential pattern response. All analyses were performed using XLStat 18 (Lumivero, USA), considering a P value <0.05.

### Health-enhancing and therapeutic effects

The overall response of the gilthead seabream intestine explant to the test ingredients was positive, with an observed modulation of the expression of most molecular markers assessed.

### Immune response to the ingredients

The explants responded to the ingredients with an upregulation of the immune-related markers included in this study (Fig. 1, 2 and 3). The IL-1β, and TNFα are cytokines that are first released upon an immune trigger (e.g. PAMPs detection in epithelium) and will boost the immune response through enhancing the secretion of other cytokines such as IL-8. The tested ingredients increased the expression of IL-1β when compared with the control, and ingredients 2b and 3b upregulated TNFα. Although these changes lack statistical significance, most likely due to the observed individual variation, ingredients 2b and 3b promoted a significantly higher expression of IL-8, indicating active immune stimulation. This chemokine promotes an inflammatory response and is released by activated monocytes and macrophages.

Here, the expression of the gene that codes for the chemokine has been evaluated, and not the active protein; however, it provides a strong insight that with the culture medium of co-fermentation of mycelium and Aurantiochytrium there is an activation of this innate immune response with possible higher levels of IL-8 that will help neutrophils and other immune cells to migrate towards the required area, in this case the gut epithelium, promoting a faster response when challenged with a pathogenic invasion. This overall immunostimulation has the exception of ingredients mainly in the lower dosage (Fig. 1, 2, 3 and Fig 4).

### Antioxidant response to the ingredients

Based on the expression of the antioxidant response-related markers, the tested ingredients had low impact (Fig. 8). The enzyme antioxidants SOD1, CAT and GPx prevent or delay the damage by oxygen radicals on tissues. Their orchestrated action arrests superoxide and oxygen radicals and neutralizes them to less or non-toxic forms such as hydrogen peroxide and, consequently, water and molecular oxygen. Glutathione peroxidase, in particular, has a relevant role by eliminating hydrogen peroxide and lipid peroxide, preventing peroxidative damage to cell membranes and others by regulating GSH (i.e. glutathione). All test products slightly increased the expression of GPx, but no significant changes were found, and with the observed level of magnitude, it does not support modulation of the antioxidant system (Fig 5, 6, 7, 9, 10, 11 and Fig. 12).

### Epithelium integrity response to the ingredients

The epithelium barrier functions are vital to avoid the entrance of pathogenic organisms, and epithelium integrity ensures regulated permeability. The tight junctions are the sealing structure of the paracellular space between adjacent cells and are constituted by a complex of proteins, among others the Tight Junction Protein 2 (TJP2), Occludin (OCL), claudins 5 and 12, the latter a pore-forming claudin. There were no significant differences in the response of explants to the different test ingredients when measured by the transcriptional modulation of the genes that code for the referred tight junction complex proteins (Fig. 9, 10,11 and Fig 12). Still, ingredient 3b tends to promote a stronger modulation of TJP2, OCL, and CLDN5, and results did not indicate dose responsiveness (Fig. 9, 10,11 and Fig 12 and Fig. 13).

An immunostimulation is evident in a dose-response manner. The best-performing ingredients were the medium resulting from mycelium co-fermentation with the lower inoculum of Aurantiochytrium (i.e. 2b-L lower dose), and the the medium resulting from mycelium co-fermentation with the higher inoculum of Aurantiochytrium (i.e. 3b-H higher dose). Overall, products performed as good or better than the commercial β-glucans.

A summary of the responses is presented in Table 6. An immunostimulation is evident in a dose-response manner. The best-performing ingredients were the medium resulting from mycelium co-fermentation with the lower inoculum of Aurantiochytrium (i.e. 2b lower dose), and the medium resulting from mycelium co-fermentation with the higher inoculum of Aurantiochytrium (i.e. 3b-H Higher dose). Overall, products performed as good or better than the commercial β-glucans.

**Table 6:**

| **Ingredients** | **Dosage** | **Immune** | **Antioxidant** | **Epithelium** |
|---|---|---|---|---|
| 2b | Low | 2 | 1.2 | 1.15 |
| | High | 2 | 1.3 | 1.08 |
| 3b | Low | 1.5 | 1.33 | 1.37 |
| | High | 2.2 | 1.15 | 1.21 |
| Beta-Glucans | Low | 1.7 | 1.1 | 1.19 |

### Conclusions

Ingredients 2b and 3b and β-glucan lead to a significant upregulation of immune processes and ingredient 2b to a downregulation of epithelium-related processes. Based on the ex-vivo screening, the tested ingredients present immunostimulatory potential and the responses are similar or, in some cases, better than the ones resulting from the utilization of a commercial product (β-glucan) used for the purpose of immune-stimulation. The tested ingredients at the applied dosages had an effect on the antioxidant response. A better immunostimulant response was achieved when a higher dose was applied.

### Second ex-vivo trial

In the second ex-vivo study conducted on Seabream fish *(Sparus aurata)* with an objective to evaluate the potential of the residual fermentation media produced from a fermentation of fungal mycelium and micro or macroalgae for their antioxidant property, immunity enhancement, epithelial integrity and mucosa-tissue regeneration in humans and fish or any livestock. The samples used for this experiment were obtained from examples 2 and 4. The samples obtained by variant 2a were by fermentation of edible fungal mycelium mentioned in example 2, while samples from variant 1a was a fermentation product of edible fungal mycelium and microalgae of disrupted cells of *Chlorella sp* derived from example 4.

The aim of this study was to evaluate the functional potential of different biomass and culture medium samples obtained from the fermentation of edible fungi mycelium, and fermentation of edible fungal mycelium with disrupted cells of Chlorella sp. A second objective of the study was to evaluate the therapeutic potential of the fermentation liquids after a controlled irritation of the intestinal mucosa using an ex vivo approach.

**Table 7:**

| **SI No** | **Samples** | **Sample code** | **Sample description** |
|---|---|---|---|
| 1 | 1a | MCSM | Freeze dried residual fermentation media obtained from fermentation of edible fungal mycelium and disrupted cells of *Chlorella sp* |
| 2 | 2a | MCLM | Freeze dried residual fermentation media obtained from fermentation of edible fungal mycelium |

### Explants

After the technical assessment and assurance of conformity of the test ingredients, these were placed in 2mL sterile Eppendorf tubes with ex vivo culture media. Two dosages were selected following fish size to simulate a 0.3% and a 1% inclusion rate in the fish feed. Ingredients were prepared immediately before the start of the intestinal explant process and were always kept at the same temperature as fish were stocked, which was the incubation temperature throughout the procedure (21°C). Previous to the trial, juvenile seabream *(Sparus aurata)* (mean body weight 17g) was fed for three weeks a standardized formulated feed to assure all nutritional requirements and health. After a preliminary assurance of good health condition, fish were captured with a soft net from stock facilities and anesthetized. Fish anterior intestines were used for the assay, which was performed following in-house standardized procedures. The response of fish intestines to the different ingredients and dosages was assessed after 5 hours. Two assays were performed: Assay #1 aimed to evaluate the functional properties of the test products through the modulation of immune and epithelial responses in the gut mucosa, relating to a potential prophylactic application of the ingredient, whereas Assay #2 was performed to assess the potential of the ingredients to attenuate an ongoing insult (irritation with H2O2), relating to a possible application as therapeutic strategy. For assay #2 the insult was inflicted by exposing the intestinal explant to a standardized dosage of H2O2 sufficient to irritate the tissue without irreversible damage to the tissue architecture integrity for one hour, after which the medium was replaced with fresh medium without the insult and the explant was treated with the ingredients.

### Sample handling

By the end of the trial, tissue samples were collected and placed in the RNALater (Invitrogen, USA) stabilizer solution until analysis. Samples were kept in a cold chain and stored at -80C upon arrival
at the laboratory.

### Molecular markers assessment

The experiment was conducted in a similar procedure as in the first ex-vivo trial with minor difference in the selection of marker genes for more specific understanding of the effect on the tissues with the treatment of the product of interest. The genes Glyceraldehyde 3-phosphate dehydrogenase (GADPH) and the 18S ribosomal subunit (18S), were used as reference genes.

Genes of interest for immune response comprised the interleukin-1beta (IL-1b), interleukin-8 (IL- 8), and tumor necrosis factor-alpha (TNF-a), cyclooxygenase 2 (cox2), immunoglobulin M (IgM) and interleukin 10 (IL-10). Genes of interest for epithelial integrity genes included occludin (OCL), tight junction protein (TJP), claudin 12 (cldn12), claudin 5 (cldn5), intestinal mucin (iMUC) and the proliferating cell nuclear antigen (PCNA). In addition, genes related to the initial stage of the wound healing process were also screened and these were hypoxia-inducible factor alpha (HIFα), integrinβ1 and vimentin. Primers for all genes were previously standardized for the tissues to assess the efficiency and correct amplification.

### Statistical analysis

Relative gene expression ratios were calculated using the Pfaffl method (Pfaffl, 2001; DOI:10.1093/nar/29.9.e45) and the control replicate of each fish for normalization. Data was log-transformed, and outlier detection was performed according to the inter quartile range (IQR) and boxplots, replacing extreme outliers (3*IQR) with the group median. Statistical analyses were conducted using both parametric, i.e., linear models followed by the pos hoc Dunnett's test, and non-parametric tests, i.e., Kruskal-Wallis followed by Dunn's test (α = 0.05). The linear models' residuals were checked for normality and homoscedasticity using diagnostic plots. Multivariate analysis was performed through Discriminant Analysis to infer groups' differential pattern response. Data are expressed as mean ± standard deviation. All analyses were performed in the R software.

### Results

To demonstrate the beneficial effects of the invention, two assays were performed:
Assay #1 aimed to evaluate the functional properties of the test products through the modulation of immune and epithelial responses in the gut mucosa, relating to a potential prophylactic application of the ingredient.
Assay #2 was performed to assess the potential of the ingredients to attenuate an ongoing insult (irritation with H₂O₂), relating to a possible application as therapeutic strategy. For assay #2 the insult was inflicted by exposing the intestinal explant to a standardized dosage of H₂O₂ sufficient to irritate the tissue without irreversible damage to the tissue architecture integrity for one hour, after which the medium was replaced with fresh medium without the insult and the explant was treated with the ingredients.

### Prophylactic application (Assay #1)

The results of functional potential analyses relating to gene expression and immune response are shown in Figures 14 to 25. Gene expression and immune-related genes have been investigated.

It could be found that there is a stronger immune response promoted by MCSM. Integrated analysis indicates that the MCSM product (i.e. derived from co-fermentation with ruptured Chlorella) present a differential response in intestinal mucosa with higher expression of immune related genes, namely the proinflammatory cytokines IL-1B and IL-8 but also the IgM. Moreover, an increase in expression of the CLDN5 and TJP2 indicates also a positive impact on the epithelium integrity. Based on the evaluated markers, the MCSM is the most promising ingredient to be applied as functional prophylactic agents.

### Therapeutic Application (Assay #2)

The results of potential therapeutic strategies after insult are shown in Figures 26 to 37.

Both types MCLM and MCSM presented immunostimulatory properties during insult. The impacts of the irritant H₂O, on the mucosa were observed with an increase in the expression of genes related to inflammatory signaling, mainly IL-8, and TNF-alpha. The MCLM and MCSM both empowered a stronger response of those and other markers indicating an active response. The integrative analysis indicates that MCLM had more impact in TNF-alpha, IgM and also in the tight junction protein 2, whereas the MCSM had higher impact in epithelium responses mainly on occluding expression. Based on the evaluated markers, the MCLM presented the capacity to increase the processes triggered in response to an insult, with a potential to promote faster recovery of the tissue. However, MCSM also seem to have a positive input when intestinal explant suffered an insult promoting a stronger immune response that will guide tissue recovery.

Table 8 shows a heatmap with mean gene expression of the evaluated processes for prophylactic and therapeutic application.

**Table 8:**

| **Group** | **Dosage** | **Immune** | **Epithelium** | **Trial** |
|---|---|---|---|---|
| Ctrl | | 1.0 | 1.0 | Prophylactic |
| Ctrl+ | | 1.62 | 1.11 | |
| MCSM | D1 | 2.49 | 1.03 | |
| | D2 | 3.35 | 1.81 | |
| MCLM | D1 | 1.82 | 1.34 | |
| | D2 | 1.44 | 1.27 | |
| H2O2 | | 6.29 | 1.44 | Therapeutic |
| Bglu | | 9.26 | 2.14 | |
| MCSM | D1 | 12.26 | 2.15 | |
| | D2 | 9.85 | 2.17 | |
| MCLM | D1 | 8.61 | 1.84 | |
| | D2 | 16.04 | 1.59 | |

### Prophylactic application (Assay #1)

The results of functional potential analyses relating to gene expression and immune response are shown in Figures 14 to 25. Gene expression and immune-related genes have been investigated.

It could be found that there is a stronger immune response promoted by MCSM. Integrated analysis indicates that the MCSM product (i.e. derived from co-fermentation with ruptured Chlorella) present a differential response in intestinal mucosa with higher expression of immune related genes, namely the proinflammatory cytokines IL-1B and IL-8 but also the IgM. Moreover, an increase in expression of the CLDN5 and TJP2 indicates also a positive impact on the epithelium integrity. Based on the evaluated markers, the MCSM is the most promising ingredient to be applied as functional prophylactic agents.

### Therapeutic Application (Assay #2)

The results of potential therapeutic strategies after insult are shown in Figures 26 to 37. Both types MCLM and MCSM presented immunostimulatory properties during insult. The impacts of the irritant H₂O₂ on the mucosa were observed with an increase in the expression of genes related to inflammatory signaling, mainly IL-8, and TNF-alpha. The MCLM and MCSM both empowered a stronger response of those and other markers indicating an active response. The integrative analysis indicates that MCLM had more impact in TNF-alpha, IgM and also in the tight junction protein 2, whereas the MCSM had higher impact in epithelium responses mainly on occluding expression. Based on the evaluated markers, the MCLM presented the capacity to increase the processes triggered in response to an insult, with a potential to promote faster recovery of the tissue. However, MCSM also seem to have a positive input when intestinal explant suffered an insult promoting a stronger immune response that will guide tissue recovery.

### References

Ahlborn, J., Stephan, A., Meckel, T., Maheshwari, G., Rühl, M. and Zorn, H., 2019. Upcycling of food industry side streams by basidiomycetes for production of a vegan protein source. International Journal of Recycling of Organic Waste in Agriculture, 8, pp.447-455. Pfaffl, 2001; DOI:10.1093/nar/29.9.e45
Cao W, Wang X, Sun S, Hu C, Zhao Y. Simultaneously upgrading biogas and purifying biogas slurry using cocultivation of Chlorella vulgaris and three different fungi under various mixed light wavelength and photoperiods. Bioresour Technol. 2017 Oct;241:701-709. PMID: 28618378).
Singh, V. et al. (2017). Microbiota dysbiosis controls the neuroinflammatory response after stroke. J Neurosci. https://doi.org/10.1523/JNEUROSCI.1114-17.2017
Trapp, T., Zajul, M., Ahlborn, J., Stephan, A., Zorn, H. and Fraatz, M.A., 2017. Submerged cultivation of Pleurotus sapidus with molasses: aroma dilution analyses by means of solid phase microextraction and stir bar sorptive extraction. Journal of Agricultural and Food Chemistry, 66(10), pp.2393-2402.
Turner, J. R. (2009). Intestinal mucosal barrier function in health and disease. Nature Reviews Immunology. https://doi.org/10.1038/nri2653

## Claims

1. A method for producing an edible product comprising:
a) cultivating edible fungal mycelium in a liquid nutrient medium under submerged fermentation conditions in a bioreactor to generate a fermentation liquid,
b) separating the fermentation liquid from the mycelium biomass after completion of fermentation by filtration or centrifugation,
c) isolating and further processing of the fermentation liquid or components thereof into the edible product.

2. The method according to claim 1 further comprising:
a) cultivating the edible fungal mycelium together with at least one microalgae or macroalgae strain,
b) separating the mycelium biomass and algae from the fermentation liquid.

3. The method according to claim 1 or 2, wherein further processing of the fermentation liquid to the edible product comprises any one of precipitation, evaporation, distillation, filtration, centrifugation, adsorption, reverse osmosis, extraction, solid-phase microextraction, chromatography, freeze-drying, spray-drying, drum-drying, electrolysis, dialysis, or combinations thereof.

4. The method according to any one of claims 1 to 3, wherein the edible fungal mycelium is derived from the phylum Basidiomycota and/or Ascomycota.

5. The method according to claim 4, wherein the edible fungal mycelium is derived from at least one fungal strain selected from the group consisting of *Pleurotus ostreatus, Pleurotus sapidus, Pleurotus pulmonarius, Ganoderma lucidum, Hericium erinaceus, Inonotus obliquus, Trametes versicolor, Grifola fondosa, Lentinula edodes, Aspergillus oryzae, Rhizopus oryzae, Fusarium graminareum, Cordyceps militaris, Cordyceps sinensis, Tuber melanosporum, Tuber magnatum, Neurospora intermedia, Neurospora sitophila*, and *Xylaria hypoxion* or combinations thereof.

6. The method according to claim 2, wherein the at least one microalgae or macroalgae strain is selected from the group consisting of *Chlorella vulgaris, Chlamydomonas reinhardtii, Haematococus pluvialis, Dunaliella salina, Scenedesmus dimorphus, Scenedesmus acuminatus, Tetraselmis suecica, Tetraselmis striatis, Arthrospira platensis, Synechococcus sp., Isochrysidus galbana, Nitzschia dissipata, Phaedactylum tricornutum, Skeletonema sp., Pseudonana sp., Odontella aurita, Schizochytrium sp., Aurantiochytrium sp., Monodus subterranea, Nannochloropsis gaditana, Nannochloropsis oceanica, Nannochloropsis oculata, Porphyridium cruentum, Pyropia yezoensis, Pyropia haitianensis, Palmaria palmata, Palmaria mollis, Chondrus crispus, Kapaphycus alvarezii, Eucheuma cottonii, Ulva lactuca, Sacharina japonica, Sacharina latissima,* and *Laminaria digitata* or combinations thereof.

7. The method according to any one of claims 1 to 6 further comprising the steps of:
a) harvesting and subjecting the algal biomass to a cell disruption treatment after cultivation, wherein the cell disruption treatment comprises any one of physical, chemical, or enzymatic treatment,
b) transferring the disrupted algal biomass to a second bioreactor containing edible fungal mycelium,
c) cultivating the edible fungal mycelium with the disrupted algal biomass in a nutrient medium under submerged fermentation conditions before separating the fermentation liquid from the mycelium biomass.

8. The method according to claim 7, wherein the nutrient medium comprises an ingredient selected from the group consisting of a carbon source, a nitrogen source, phosphates, sulphates, and/or minerals.

9. The method according to any one of claims 1 to 8, wherein the fermentation is performed as a batch, fed-batch or continuous process.

10. The method according to any one of claims 1 to 9, wherein the edible product is an animal feed, feed supplement, human food supplement and/or drug.

11. An edible product that is based on fermentation liquid produced by cultivating edible fungal mycelium with or without microalgae or macroalgae in a liquid nutrient medium, wherein the edible product is obtainable by a method according to any one of claims 1 to 10.

12. The product according to claim 11, further comprising one or more additives selected from the group consisting of minerals, vitamins, and/or probiotic microorganisms.

13. An edible product obtainable by a method according to any one of claims 1 to 10 for use as a drug.

14. An edible product obtainable by a method according to any one of claims 1 to 10 for use as a feed supplement or food supplement.

15. An edible product obtainable by a method according to any one of claims 1 to 10 for use in the prevention or treatment of a disease or condition that is associated with impaired immune response, impaired epithelial integrity or mucosa tissue damage.

16. The product according to claim 15, wherein the diseases or conditions that are associated with an impaired immune response, impaired epithelial integrity or mucosa tissue damage are any one of immunodeficiency disorders, autoimmune diseases, chronic inflammatory diseases, infectious diseases, inflammatory bowel disease (IBD), celiac disease, asthma, allergic rhinitis, chronic obstructive pulmonary disease (COPD), respiratory infections, atopic dermatitis, cystic fibrosis, psoriasis, graft-versus-host disease, necrotizing enterocolitis (NEC), sepsis or cancer.
